(19)

Europäisches
Patentamt
European
Patent Office
Office européen
des brevets

(11) **EP 2 316 930 A1**

(12) **EUROPEAN PATENT APPLICATION**

(43) Date of publication:
04.05.2011 Bulletin 2011/18

(51) Int Cl.:
*C12N 9/64* *(2006.01)*    *A61K 38/36* *(2006.01)*

(21) Application number: **10181064.6**

(22) Date of filing: **14.09.2006**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **14.09.2005 EP 05108433
18.07.2006 EP 06117378**

(62) Document number(s) of the earlier application(s) in
accordance with Art. 76 EPC:
**06793524.7 / 1 926 817**

(71) Applicant: **Novo Nordisk Health Care AG
8050 Zürich (CH)**

(72) Inventors:
• **Østergaard, Henrik
3650 Ølstykke (DK)**

• **Olsen, Ole, Hvilsted
2700 Brønshøj (DK)**
• **Larsen, Katrine, Skaarup
2000 Frederiksberg (DK)**
• **Stennicke, Henning
2980 Kokkedal (DK)**

(74) Representative: **Owald, Micael et al
Novo Nordisk A/S
Corporate Patents
Novo Allé
2880 Bagsvaerd (DK)**

Remarks:
This application was filed on 28-09-2010 as a
divisional application to the application mentioned
under INID code 62.

(54) **Human coagulation factor VII polypeptides**

(57)    The present invention relates to a novel human
coagulation Factor VIIa variant having a substitution at
position 286 in SEQ ID NO:1 and coagulant activity as
well as polynucleotide constructs encoding such vari-
ants, vectors and host cells comprising and expressing
the polynucleotide, pharmaceutical compositions, uses
and methods of treatment.

EP 2 316 930 A1

**Description**

**FIELD OF THE INVENTION**

The present invention relates to novel human coagulation Factor VIIa polypeptides having coagulant activity as well as polynucleotide constructs encoding such polypeptides, vectors and host cells comprising and expressing the polynucleotide, pharmaceutical compositions, uses and methods of treatment.

**BACKGROUND OF THE INVENTION**

Blood coagulation is a process consisting of a complex interaction of various blood components (or factors) that eventually gives rise to a fibrin clot. Generally, the blood components, which participate in what has been referred to as the coagulation "cascade", are enzymatically inactive proteins (proenzymes or zymogens) that are converted to proteolytic enzymes by the action of an activator (which itself is an activated clotting factor). Coagulation factors that have undergone such a conversion are generally referred to as "active factors", and are designated by the addition of the letter "a" to the name of the coagulation factor (e.g. Factor VIIa).

Initiation of the haemostatic process is mediated by the formation of a complex between tissue factor, exposed as a result of injury to the vessel wall, and Factor VIIa. This complex then converts Factors IX and X to their active forms. Factor Xa converts limited amounts of prothrombin to thrombin on the tissue factor-bearing cell. Thrombin activates platelets and Factors V and VIII into Factors Va and VIIIa, both cofactors in the further process leading to the full thrombin burst. This process includes generation of Factor Xa by Factor IXa (in complex with factor VIIIa) and occurs on the surface of activated platelets. Thrombin finally converts fibrinogen to fibrin resulting in formation of a fibrin clot. In recent years Factor VII and tissue factor have been found to be the main initiators of blood coagulation.

Factor VII is a trace plasma glycoprotein that circulates in blood as a single-chain zymogen. The zymogen is catalytically inactive. Single-chain Factor VII may be converted to two-chain Factor VIIa by Factor Xa, Factor XIIa, Factor IXa, Factor VIIa or thrombin in vitro. Factor Xa is believed to be the major physiological activator of Factor VII. Like several other plasma proteins involved in haemostasis, Factor VII is dependent on Vitamin K for its activity, which is required for the gamma-carboxylation of multiple glutamic acid residues that are clustered close to the amino terminus of the protein. These gamma-carboxylated glutamic acids are required for the metal ion-induced interaction of Factor VII with phospholipids. The conversion of zymogen Factor VII into the activated two-chain molecule occurs by cleavage of an internal $Arg_{152}$-$Ile_{153}$ peptide bond. In the presence of tissue factor, phospholipids and calcium ions, the two-chain Factor VIIa rapidly activates Factor X or Factor IX by limited proteolysis.

Blood coagulation is regulated by several inhibitors found in human plasma which inhibit coagulation and fibrinolytic enzymes thereby restricting thrombosis and fibrinolysis to the site of vessel injury. This is very important to avoid systemic coagulation and fibrin formation. The FVIIa-TF complex and FXa can be inhibited by tissue factor pathway inhibitor (TFPI) which is a protein composed of three Kunitz domains. Inhibition by TFPI only allows the tissue factor initiated pathway to generate small amounts of thrombin insufficient to produce fibrin. The inhibition by TFPI occurs in two steps. First, TFPI forms a complex with free FXa and then the TFPI-FXa complex binds to the FVIIa-TF complex forming a quarternary inhibitory complex.

ATIII also plays an important physiological role being able to inhibit several coagulation factors with the main targets being FXa and thrombin but also the FVIIa-TF complex. ATIII is a very abundant inhibitor having a concentration of 2.5 $\mu$M in human plasma. The activity of ATIII is greatly enhanced by sulphated glycoaminoglycans lining the vascular walls.

It is often desirable to stimulate or improve the coagulation cascade in a subject. Factor VIIa has been used to control bleeding disorders that have several causes such as clotting factor deficiencies (e.g. haemophilia A and B or deficiency of coagulation Factors XI or VII) or clotting factor inhibitors. Factor VIIa has also been used to control excessive bleeding occurring in subjects with a normally functioning blood clotting cascade (no clotting factor deficiencies or inhibitors against any of the coagulation factors). Such bleeding may, for example, be caused by a defective platelet function, thrombocytopenia or von Willebrand's disease. Bleeding is also a major problem in connection with surgery and other forms of tissue damage.

European Patent No. 200,421 (ZymoGenetics) relates to the nucleotide sequence encoding human Factor VII and the recombinant expression of Factor VII in mammalian cells.

Dickinson et al. (Proc. Natl. Acad. Sci. USA (1996) 93, 14379-14384) relates to the identification of surface residues mediating tissue factor binding and catalytic function of the serine protease factor VIIa.

There is a need for variants of Factor VIIa having coagulant activity, variants with high activity that can be administered at relatively low doses, variants which are more resistant to inactivation by endogenous inhibitors, and variants which do not produce the undesirable side effects such as systemic activation of the coagulation system and bleeding.

## SUMMARY OF THE INVENTION

In a broad aspect the present invention relates to Factor VIIa polypeptides exhibiting increased functional in vivo half-life as compared to human wild-type Factor VIIa.

In one broad aspect the present invention relates to Factor VIIa polypeptides exhibiting increased resistance to inactivation by endogenous inhibitors.

In a first aspect, the invention relates to a Factor VII polypeptide comprising one or more substitutions relative to the amino acid sequence of SEQ ID NO: 1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide has increased functional in vivo half-life as compared to human wild-type Factor VIIa.

In a second aspect, the invention relates to a polynucleotide construct encoding a Factor VII polypeptide comprising one or more substitutions relative to the amino acid sequence of SEQ ID NO:1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide has increased functional in vivo half-life as compared to human wild-type Factor VIIa.

In a third aspect, the invention relates to a host cell comprising a polynucleotide construct encoding a Factor VII polypeptide comprising one or more substitutions relative to the amino acid sequence of SEQ ID NO: 1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide has increased functional in vivo half-life as compared to human wild-type Factor VIIa.

In a further aspect, the invention relates to a transgenic animal containing and expressing the polynucleotide construct encoding a Factor VII polypeptide comprising one or more substitutions relative to the amino acid sequence of SEQ ID NO: 1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide has increased functional in vivo half-life as compared to human wild-type Factor VIIa

In a further aspect, the invention relates to a transgenic plant containing and expressing the polynucleotide construct encoding a Factor VII polypeptide comprising one or more substitutions relative to the amino acid sequence of SEQ ID NO: 1, wherein the substitutions are replacement with any other amino acid of one or more amino acids at a position selected from the group consisting of position 172, 173,175,176,177,196,197,198,199,200, 203,235,237,238,239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide has increased functional in vivo half-life as compared to human wild-type Factor VIIa.

In a further aspect, the invention relates to a method for producing the Factor VII polypeptide comprising one or more substitutions relative to the amino acid sequence of SEQ ID NO:1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide has increased functional in vivo half-life as compared to human wild-type Factor VIIa, the method comprising cultivating a cell as defined in any one of claims 65-68 in an appropriate growth medium under conditions allowing expression of the polynucleotide construct and recovering the resulting polypeptide from the culture medium.

In a further aspect, the invention relates to a method for producing the Factor VII polypeptide comprising one or more substitutions relative to the amino acid sequence of SEQ ID NO:1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide has increased functional in vivo half-life as compared to human wild-type Factor VIIa, the method comprising recovering the Factor VII polypeptide from milk produced by the transgenic animal according to the invention.

In a further aspect, the invention relates to a method for producing the Factor VII polypeptide comprising one or more substitutions relative to the amino acid sequence of SEQ ID NO:1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide has increased functional in vivo half-life as compared to human wild-type Factor VIIa, the method comprising cultivating a cell of a transgenic plant according to the invention, and recovering the Factor VII polypeptide from the plant.

In a further aspect, the invention relates to a pharmaceutical composition comprising a Factor VII polypeptide comprising one or more substitutions relative to the amino acid sequence of SEQ ID NO: 1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide has increased functional in vivo half-life as compared to human wild-type Factor VIIa; and, optionally, a pharmaceutically acceptable carrier.

In a further aspect, the invention relates to the use of a Factor VII polypeptide comprising one or more substitutions relative to the amino acid sequence of SEQ ID NO: 1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide has increased functional in vivo half-life as compared to human wild-type Factor VIIa; for the preparation of a medicament for the treatment of bleeding disorders or bleeding episodes or for the enhancement of the normal haemostatic system.

In a further aspect, the invention relates to a method for the treatment of bleeding disorders or bleeding episodes in a subject or for the enhancement of the normal haemostatic system, the method comprising administering a therapeutically or prophylactically effective amount of a Factor VII polypeptide comprising one or more substitutions relative to the amino acid sequence of SEQ ID NO: 1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide has increased functional in vivo half-life as compared to human wild-type Factor VIIa; to a subject in need thereof.

In a further aspect, the invention relates to a Factor VII polypeptide comprising one or more substitutions relative to the amino acid sequence of SEQ ID NO: 1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide has increased functional in vivo half-life as compared to human wild-type Factor VIIa; for use as a medicament.

In a further aspect, the invention relates to a Factor VII polypeptide comprising one or more substitutions relative to the amino acid sequence of SEQ ID NO: 1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide exhibits increased resistance to inactivation by an endogenous inhibitor of the FVII polypeptide relative to wild-type human FVIIa.

In a further aspect, the invention relates to a pharmaceutical composition comprising a Factor VII polypeptide comprising one or more substitutions relative to the amino acid sequence of SEQ ID NO:1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide exhibits increased resistance to inactivation by an endogenous inhibitor of the FVII polypeptide relative to wild-type human FVIIa; and, optionally, a pharmaceutically acceptable carrier.

In a further aspect, the invention relates to the use of a Factor VII polypeptide comprising one or more substitutions relative to the amino acid sequence of SEQ ID NO: 1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide exhibits increased resistance to inactivation by an endogenous inhibitor of the FVII polypeptide relative to wild-type human FVIIa; for the preparation of a medicament for the treatment of bleeding disorders or bleeding episodes or for the enhancement of the normal haemostatic system.

In a further aspect, the invention relates to a method for the treatment of bleeding disorders or bleeding episodes in a subject or for the enhancement of the normal haemostatic system, the method comprising administering a therapeutically or prophylactically effective amount of a Factor VII polypeptide comprising one or more substitutions relative to the amino acid sequence of SEQ ID NO: 1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide exhibits increased resistance to inactivation by an endogenous inhibitor of the FVII polypeptide relative to wild-type human FVIIa; to a subject in need thereof.

In one embodiment the Factor VII polypeptide according to the invention is not Q366E-FVII.

In one embodiment the Factor VII polypeptide according to the invention is not Q366A-FVII.

In one embodiment the Factor VII polypeptide according to the invention is not Q366G-FVII.

## DETAILED DESCRIPTION OF THE INVENTION

A distinctive feature of serine proteases, and proteases in general, is their substrate specificity. For small substrates, like peptides, specificity is generally dictated by the complementary features of the active-site cleft and the peptidyl sequence surrounding the scissile bond. A feature which also seems to hold true for the recognition of inhibitors belonging to the Kunitz-type and serine protease inhibitor (Serpin) families of proteins, presenting a bait loop to their cognate proteases as a part of the inhibition mechanism. In contrast, proteolytic processing of larger substrates are often strongly influenced by interactions outside the active-site region, at so-called exosites. One example is the proteolytic activation of coagulation factor X by the tissue factor-factor VIIa complex, for which it has been shown that macromolecular substrate binding to the binary complex occurs independently of scissile bond docking (Shobe et al. (1999) J.Biol.Chem., 274, 24171-24175, Baugh et al. (2000) J.Biol.Chem., 275, 28826-28833).

The distinct modes of macromolecular substrate and inhibitor recognition allows for the design of factor VIIa active-site variants exhibiting increased resistance to inactivation by endogenous inhibitors, such as anti-thrombin III or tissue factor pathway inhibitor (TFPI), while maintaining their ability to induce coagulation through exosite-driven macromolecular substrate activation.

Thus, the present invention relates to the design and use of Factor VIIa polypeptides with reduced susceptibility to inhibitor inactivation as substitutes for recombinant wild type factor VIIa. In one embodiment these novel FVII polypeptides has a retained activity but are less prone to inhibition by circulating serpins, such as ATIII found in human plasma. Such novel FVII polypeptides may have an extended functional half-life in blood and thereby be superior to native rFVIIa in a long-term treatment of patients with severe bleeding disorders.

It is suggested by the inventors of the present invention, that human coagulation Factor VIIa polypeptide variants, wherein the amino acids of position independently selected from the group consisting 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 have been replaced by any other amino acids, are more resistant to inactivation by endogenous inhibitors as compared to wild type human coagulation Factor VIIa.

The term "any other amino acid" as used herein means one amino acid that are different from that amino acid naturally present at that position. This includes but are not limited to amino acids that can be encoded by a polynucleotide. In one embodiment the different amino acid is in natural L-form and can be encoded by a polynucleotide. A specific example being L-cysteine (Cys).

The term "activity" as used herein means the ability of a Factor VII polypeptide to convert its substrate Factor X to the active Factor Xa. The activity of a Factor VII polypeptide may be measured with the *"In Vitro* Proteolysis Assay" (see Example 5).

Due to the higher resistance to inhibition of the described Factor VIIa polypeptide variants compared to native FVIIa, a lower dose may be adequate to obtain a functionally adequate concentration at the site of action and thus it will be possible to administer a lower dose and/or with lower frequency to the subject having bleeding episodes or needing enhancement of the normal haemostatic system.

It has been found by the inventors of the present invention that by replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO: 1, the Factor VII polypeptide will obtain higher resistance to endogenous proteases. Such Factor VIIa polypeptide variants may exhibit an increased half-life, which may be therapeutically useful in situations where a longer lasting procoagulant activity is wanted.

In one embodiment of the invention, the Factor VIIa polypeptides exhibits increased resistance to inactivation by an endogenous inhibitor of the FVII polypeptide relative to wild-type human FVIIa, wherein the ratio between the inhibition of the Factor VII polypeptide and the inhibition of wild type FVIIa is less than about 1.00, such as less than about 0.9, such as less than about 0.8, such as less than about 0.7, such as less than about 0.6, such as less than about 0.5, such as less than about 0.4.

In one embodiment of the invention, the Factor VIIa polypeptides exhibits increased resistance to inactivation by an endogenous inhibitor of the FVII polypeptide relative to wild-type human FVIIa, wherein the inhibitor is of kunitz-type, such as tissue factor pathway inhibitor (TFPI).

In one embodiment of the invention, the Factor VIIa polypeptides exhibits increased resistance to inactivation by an endogenous inhibitor of the FVII polypeptide relative to wild-type human FVIIa, wherein the inhibitor is a serpin such as anti-thrombin III.

In one embodiment of the invention, the Factor VIIa polypeptides exhibits increased resistance to inactivation by an endogenous inhibitor of the FVII polypeptide relative to wild-type human FVIIa, wherein the inhibitor is alpha-2-macroglobulin.

In one embodiment of the invention, the Factor VIIa polypeptides exhibits an increased half-life due to resistance to inactivation by inhibitors, such as endogenous protease inhibitors. In one embodiment this endogenous inhibitor is selected from anti-thrombin III and tissue factor pathway inhibitor (TFPI).

In one embodiment additional replacement of amino acids facilitate formation of a more active conformation of the molecule, thereby providing a FVIIa polypeptide with higher intrinsic activity.

In a further embodiment of the invention, the Factor VII polypeptide is a polypeptide wherein the Factor VII polypeptide has increased functional in vivo half-life relative to human wild-type Factor VIIa.

In a further embodiment of the invention, the Factor VII polypeptide is a polypeptide wherein A175 is replaced with any other amino acid.

In a further embodiment of the invention, the Factor VII polypeptide is a polypeptide wherein Q176 is replaced with any other amino acid.

In a further embodiment of the invention, the Factor VII polypeptide is a polypeptide wherein L177 is replaced with any other amino acid.

In a further embodiment of the invention, the Factor VII polypeptide is a polypeptide wherein D196 is replaced with any other amino acid.

In a further embodiment of the invention, the Factor VII polypeptide is a polypeptide wherein K197 is re-placed with any other amino acid.

In a further embodiment of the invention, the Factor VII polypeptide is a polypeptide wherein I198 is re-placed with any other amino acid.

In a further embodiment of the invention, the Factor VII polypeptide is a polypeptide wherein K199 is re-placed with any other amino acid.

In a further embodiment of the invention, the Factor VII polypeptide is a polypeptide wherein G237 is re-placed with any other amino acid.

In a further embodiment of the invention, the Factor VII polypeptide is a polypeptide wherein T238 is replaced with any other amino acid.

In a further embodiment of the invention, the Factor VII polypeptide is a polypeptide wherein T239 is replaced with any other amino acid.

In a further embodiment of the invention, the Factor VII polypeptide is a polypeptide wherein Q286 is replaced with any other amino acid.

In a further embodiment of the invention, the Factor VII polypeptide is a polypeptide wherein L287 is replaced with any other amino acid.

In a further embodiment of the invention, the Factor VII polypeptide is a polypeptide wherein L288 is replaced with any other amino acid.

In a further embodiment of the invention, the Factor VII polypeptide is a polypeptide wherein D289 is replaced with any other amino acid.

In a further embodiment of the invention, the Factor VII polypeptide is a polypeptide wherein R290 is replaced with any other amino acid.

In a further embodiment of the invention, the Factor VII polypeptide is a polypeptide wherein G291 is replaced with any other amino acid.

In a further embodiment of the invention, the Factor VII polypeptide is a polypeptide wherein A292 is replaced with any other amino acid.

In a further embodiment of the invention, the Factor VII polypeptide is a polypeptide wherein T293 is replaced with any other amino acid.

In a further embodiment of the invention, the Factor VII polypeptide is a polypeptide wherein A294 is replaced with any other amino acid.

In a further embodiment of the invention, the Factor VII polypeptide is a polypeptide wherein L295 is replaced with any other amino acid.

In a further embodiment of the invention, the Factor VII polypeptide is a polypeptide wherein L297 is replaced with any other amino acid.

In a further embodiment of the invention, the Factor VII polypeptide is a polypeptide wherein V299 is replaced with any other amino acid.

In a further embodiment of the invention, the Factor VII polypeptide is a polypeptide wherein M327 is replaced with any other amino acid.

In a further embodiment of the invention, the Factor VII polypeptide is a polypeptide wherein K341 is replaced with any other amino acid.

In a further embodiment of the invention, the Factor VII polypeptide is a polypeptide wherein S363 is replaced with any other amino acid.

In a further embodiment of the invention, the Factor VII polypeptide is a polypeptide wherein W364 is replaced with any other amino acid.

In a further embodiment of the invention, the Factor VII polypeptide is a polypeptide wherein G365 is replaced with any other amino acid.

In a further embodiment of the invention, the Factor VII polypeptide is a polypeptide wherein Q366 is replaced with any other amino acid. In one embodiment the Factor VII polypeptide according to the invention is not a Factor VII polypeptide selected from the list consisting of Q366E-FVII, Q366A-FVII, and Q366G-FVII.

In a further embodiment of the invention, the Factor VII polypeptide is a polypeptide wherein V172 is replaced with any other amino acid.

In a further embodiment of the invention, the Factor VII polypeptide is a polypeptide wherein N173 is replaced with any other amino acid.

In a further embodiment of the invention, the Factor VII polypeptide is a polypeptide wherein N200 is replaced with any other amino acid.

In a further embodiment of the invention, the Factor VII polypeptide is a polypeptide wherein N203 is replaced with any other amino acid.

In a further embodiment of the invention, the Factor VII polypeptide is a polypeptide wherein V235 is replaced with any other amino acid.

In a further embodiment of the invention, the Factor VII polypeptide is a polypeptide wherein N240 is replaced with any other amino acid.

In a further embodiment of the invention, the Factor VII polypeptide is a polypeptide wherein D319 is replaced with any other amino acid.

In a further embodiment of the invention, the Factor VII polypeptide is a polypeptide wherein S320 is replaced with any other amino acid.

In a further embodiment of the invention, the Factor VII polypeptide is a polypeptide wherein P321 is replaced with any other amino acid.

In a further embodiment of the invention, the Factor VII polypeptide is a polypeptide wherein G367 is replaced with any other amino acid.

In a further embodiment of the invention, the Factor VII polypeptide is a polypeptide wherein T370 is replaced with any other amino acid.

In a further embodiment of the invention, the Factor VII polypeptide is a polypeptide wherein H373 is replaced with any other amino acid.

In a further embodiment of the invention, the Factor VII polypeptide is a polypeptide wherein G237 is replaced with any other amino acid selected from Ala, Val, Leu, Ile, Phe, Met, Trp, Tyr, Asp, Asn, Glu, Gln, His, Lys, Arg, Cys, Thr, Ser.

In a further embodiment of the invention, the Factor VII polypeptide is a polypeptide wherein T238 is replaced with any other amino acid selected from Ala, Gly, Val, Leu, Ile, Phe, Met, Trp, Tyr, Asp, Asn, Glu, Gln, His, Lys, Arg, Cys, Ser.

In a further embodiment of the invention, the Factor VII polypeptide is a polypeptide wherein T239 is replaced with any other amino acid selected from Gly, Ala, Val, Leu, Ile, Phe, Met, Trp, Tyr, Asp, Asn, Glu, Gln, His, Lys, Arg, Cys, Ser.

In a further embodiment of the invention, the Factor VII polypeptide is a polypeptide wherein Q286 is replaced with any other amino acid selected from Gly, Ala, Val, Leu, Ile, Phe, Met, Trp, Tyr, Asp, Asn, Glu, His, Lys, Arg, Cys, Thr, Ser.

In a further embodiment of the invention, the Factor VII polypeptide is a polypeptide wherein L287 is replaced with any other amino acid selected from Gly, Ala, Val, Ile, Phe, Met, Trp, Tyr, Asp, Asn, Glu, Gln, His, Lys, Arg, Cys, Thr, Ser.

In a further embodiment of the invention, the Factor VII polypeptide is a polypeptide wherein L287 is replaced with any other amino acid selected from Thr and Ser.

In a further embodiment of the invention, the Factor VII polypeptide is a polypeptide wherein L288 is replaced with any other amino acid selected from Gly, Ala, Val, Ile, Phe, Met, Trp, Tyr, Asp, Asn, Glu, Gln, His, Lys, Arg, Cys, Thr, Ser.

In a further embodiment of the invention, the Factor VII polypeptide is a polypeptide wherein D289 is replaced with any other amino acid selected from Gly, Ala, Val, Leu, Ile, Phe, Met, Trp, Tyr, Asn, Glu, Gln, His, Lys, Arg, Cys, Ser, Thr.

In a further embodiment of the invention, the Factor VII polypeptide is a polypeptide wherein R290 is replaced with any other amino acid selected from Gly, Ala, Val, Leu, Ile, Phe, Met, Trp, Tyr, Asp, Asn, Glu, Gln, His, Lys, Cys, Thr, Ser.

In a further embodiment of the invention, the Factor VII polypeptide is a polypeptide wherein A292 is replaced with any other amino acid selected from Gly, Val, Leu, Ile, Phe, Met, Trp, Tyr, Asp, Asn, Glu, Gln, His, Lys, Arg, Cys, Thr, Ser.

In a further embodiment of the invention, the Factor VII polypeptide is a polypeptide wherein T293 is replaced with any other amino acid selected from Gly, Ala, Val, Leu, Ile, Phe, Met, Trp, Tyr, Asp, Asn, Glu, Gln, His, Lys, Arg, Cys, Ser.

In a further embodiment of the invention, the Factor VII polypeptide is a polypeptide wherein A294 is replaced with any other amino acid selected from Gly, Val, Leu, Ile, Phe, Met, Trp, Tyr, Asp, Asn, Glu, Gln, His, Lys, Arg, Cys, Thr, Ser.

In a further embodiment of the invention, the Factor VII polypeptide is a polypeptide wherein A294 is replaced with any other amino acid selected from Thr and Ser.

In a further embodiment of the invention, the Factor VII polypeptide is a polypeptide wherein L295 is replaced with any other amino acid selected from Gly, Ala, Val, Ile, Phe, Met, Trp, Tyr, Asp, Asn, Glu, Gln, His, Lys, Arg, Cys, Thr, Ser.

In a further embodiment of the invention, the Factor VII polypeptide is a polypeptide wherein L297 is replaced with any other amino acid selected from Gly, Ala, Val, Ile, Phe, Met, Trp, Tyr, Asp, Asn, Glu, Gln, His, Lys, Arg, Cys, Thr, Ser.

In a further embodiment of the invention, the Factor VII polypeptide is a polypeptide wherein V299 is replaced with any other amino acid selected from Gly, Ala, Leu, Ile, Phe, Met, Trp, Tyr, Asp, Asn, Glu, Gln, His, Lys, Arg, Cys, Thr, Ser.

In a further embodiment of the invention, the Factor VII polypeptide is a polypeptide wherein M327 is replaced with any other amino acid selected from Gly, Ala, Val, Leu, Ile, Phe, Trp, Tyr, Asp, Asn, Glu, Gln, His, Lys, Arg, Cys, Ser, Thr.

In a further embodiment of the invention, the Factor VII polypeptide is a polypeptide wherein K341 is replaced with any other amino acid selected from Gly, Ala, Val, Leu, Ile, Phe, Met, Trp, Tyr, Asp, Asn, Glu, Gln, His, Arg, Cys, Ser, Thr.

In a further embodiment of the invention, the Factor VII polypeptide is a polypeptide wherein S363 is replaced with any other amino acid selected from Gly, Ala, Val, Leu, Ile, Phe, Met, Trp, Tyr, Asp, Asn, Glu, Gln, His, Lys, Arg, Cys, Thr.

In a further embodiment of the invention, the Factor VII polypeptide is a polypeptide wherein W364 is replaced with any other amino acid selected from Gly, Ala, Val, Leu, Ile, Phe, Met, Tyr, Asp, Asn, Glu, Gln, His, Lys, Arg, Cys, Thr, Ser.

In a further embodiment of the invention, the Factor VII polypeptide is a polypeptide wherein G365 is replaced with any other amino acid selected from Ala, Val, Leu, Ile, Phe, Met, Trp, Tyr, Asp, Asn, Glu, Gln, His, Lys, Arg, Cys, Thr, Ser.

In a further embodiment of the invention, the Factor VII polypeptide is a polypeptide wherein Q366 is replaced with any other amino acid selected from Gly, Ala, Val, Leu, Ile, Phe, Met, Trp, Tyr, Asp, Asn, Glu, His, Lys, Arg, Cys, Thr, Ser.

In a further embodiment of the invention, the Factor VII polypeptide is a polypeptide wherein Q366 is replaced with any other amino acid selected from Val, Leu, Ile, Phe, Met, Trp, Tyr, Asp, Asn, His, Lys, Arg, Cys, Thr, Ser.

In a further embodiment of the invention, the Factor VII polypeptide is a polypeptide wherein Q366 is replaced with any other amino acid selected from Gly, Ala, Glu.

In a further embodiment of the invention, the Factor VII polypeptide is a polypeptide wherein V172 is replaced with any other amino acid selected from Gly, Ala, Leu, Ile, Phe, Met, Trp, Tyr, Asp, Asn, Glu, Gln, His, Lys, Arg, Cys, Ser, Thr.

In a further embodiment of the invention, the Factor VII polypeptide is a polypeptide wherein N173 is replaced with any other amino acid selected from Gly, Ala, Val, Leu, Ile, Phe, Met, Trp, Tyr, Asp, Glu, Gln, His, Lys, Arg, Cys, Ser, Thr.

In a further embodiment of the invention, the Factor VII polypeptide is a polypeptide wherein A175 is replaced with any other amino acid selected from Gly, Leu, Ile, Phe, Met, Trp, Tyr, Asp, Asn, Glu, Gln, His, Lys, Arg, Cys, Ser, Val, Thr.

In a further embodiment of the invention, the Factor VII polypeptide is a polypeptide wherein Q176 is replaced with any other amino acid selected from Gly, Ala, Leu, Ile, Phe, Met, Trp, Tyr, Asp, Asn, Glu, His, Lys, Arg, Cys, Ser, Val, Thr.

In a further embodiment of the invention, the Factor VII polypeptide is a polypeptide wherein L177 is replaced with any other amino acid selected from Gly, Ala, Ile, Phe, Met, Trp, Tyr, Asp, Asn, Glu, Gln, His, Lys, Arg, Cys, Ser, Val, Thr.

In a further embodiment of the invention, the Factor VII polypeptide is a polypeptide wherein D196 is replaced with any other amino acid selected from Gly, Ala, Leu, Ile, Phe, Met, Trp, Tyr, Asn, Glu, Gln, His, Lys, Arg, Cys, Ser, Val, Thr.

In a further embodiment of the invention, the Factor VII polypeptide is a polypeptide wherein K197 is replaced with any other amino acid selected from Gly, Ala, Leu, Ile, Phe, Met, Trp, Tyr, Asp, Asn, Glu, Gln, His, Arg, Cys, Ser, Val, Thr.

In a further embodiment of the invention, the Factor VII polypeptide is a polypeptide wherein I198 is replaced with any other amino acid selected from Gly, Ala, Leu, Phe, Met, Trp, Tyr, Asp, Asn, Glu, Gln, His, Lys, Arg, Cys, Ser, Val, Thr.

In a further embodiment of the invention, the Factor VII polypeptide is a polypeptide wherein K199 is replaced with any other amino acid selected from Gly, Ala, Leu, Ile, Phe, Met, Trp, Tyr, Asp, Asn, Glu, Gln, His, Arg, Cys, Ser, Val, Thr.

In a further embodiment of the invention, the Factor VII polypeptide is a polypeptide wherein N200 is replaced with any other amino acid selected from Gly, Ala, Val, Leu, Ile, Phe, Met, Trp, Tyr, Asp, Glu, Gln, His, Lys, Arg, Cys, Ser, Thr.

In a further embodiment of the invention, the Factor VII polypeptide is a polypeptide wherein N203 is replaced with any other amino acid selected from Gly, Ala, Val, Leu, Ile, Phe, Met, Trp, Tyr, Asp, Glu, Gln, His, Lys, Arg, Cys, Ser, Thr.

In a further embodiment of the invention, the Factor VII polypeptide is a polypeptide wherein V235 is replaced with any other amino acid selected from Gly, Ala, Leu, Ile, Phe, Met, Trp, Tyr, Asp, Asn, Glu, Gln, His, Lys, Arg, Cys, Ser, Thr.

In a further embodiment of the invention, the Factor VII polypeptide is a polypeptide wherein N240 is replaced with any other amino acid selected from Gly, Ala, Val, Leu, Ile, Phe, Met, Trp, Tyr, Asp, Glu, Gln, His, Lys, Arg, Cys, Ser, Thr.

In a further embodiment of the invention, the Factor VII polypeptide is a polypeptide wherein D319 is replaced with any other amino acid selected from Gly, Ala, Val, Leu, Ile, Phe, Met, Trp, Tyr, Asn, Glu, Gln, His, Lys, Arg, Cys, Ser, Thr.

In a further embodiment of the invention, the Factor VII polypeptide is a polypeptide wherein S320 is replaced with any other amino acid selected from Gly, Ala, Val, Leu, Ile, Phe, Met, Trp, Tyr, Asp, Asn, Glu, Gln, His, Lys, Arg, Cys, Thr.

In a further embodiment of the invention, the Factor VII polypeptide is a polypeptide wherein P321 is replaced with any other amino acid selected from Gly, Ala, Val, Leu, Ile, Phe, Met, Trp, Tyr, Asp, Asn, Glu, Gln, His, Lys, Arg, Cys, Ser, Thr.

In a further embodiment of the invention, the Factor VII polypeptide is a polypeptide wherein G367 is replaced with any other amino acid selected from Ala, Val, Leu, Ile, Phe, Met, Trp, Tyr, Asp, Asn, Glu, Gln, His, Lys, Arg, Cys, Ser, Thr.

In a further embodiment of the invention, the Factor VII polypeptide is a polypeptide wherein T370 is replaced with any other amino acid selected from Gly, Ala, Val, Leu, Ile, Phe, Met, Trp, Tyr, Asp, Asn, Glu, Gln, His, Lys, Arg, Cys, Ser.

In a further embodiment of the invention, the Factor VII polypeptide is a polypeptide wherein H373 is replaced with any other amino acid selected from Gly, Ala, Val, Leu, Ile, Phe, Met, Trp, Tyr, Asp, Asn, Glu, Gln, Lys, Arg, Cys, Ser, Thr.

In a further embodiment of the invention, the Factor VII polypeptide is a polypeptide wherein i) L287 is replaced with any other amino acid selected from Thr and Ser and ii) A294 is replaced with any other amino acid selected from Thr and Ser, and iii) M298 has been replaced by a Lys.

In a further embodiment of the invention, the Factor VII polypeptide is a polypeptide wherein i) L287 is replaced with any other amino acid selected from Thr and Ser and ii) A294 is replaced with any other amino acid selected from Thr and Ser, and iii) M298 has been replaced by a Lys, and iv) E296 has been replaced by an amino acid selected from the group consisting of Ile, Leu, Thr and Val.

In a further embodiment of the invention, the Factor VII polypeptide is a polypeptide wherein i) L287 is replaced with any other amino acid selected from Thr and Ser and ii) A294 is replaced with any other amino acid selected from Thr and Ser, and iii) M298 has been replaced by a Lys, and iv) V158 has been replaced by an amino acid selected from the group consisting of Asp and Glu.

In a further embodiment of the invention, the Factor VII polypeptide is a polypeptide wherein i) L287 is replaced with any other amino acid selected from Thr and Ser and ii) A294 is replaced with any other amino acid selected from Thr and Ser, and iii) M298 has been replaced by a Lys, and iv) E296 has been replaced by an amino acid selected from the group consisting of Ile, Leu, Thr and Val, and v) V158 has been replaced by an amino acid selected from the group consisting of Asp and Glu.

In a further embodiment of the invention, the Factor VII polypeptide is a polypeptide selected from the group consisting of:

V158D/L287T/A294S/E296I/M298K-FVIIA;      V158D/L287T/A294S/E296V/M298K-FVIIA;
V158D/L287T/A294S/E296L/M298K-FVIIA;
V158D/L287T/A294S/E296T/M298K-FVIIA;      V158D/L287T/A294T/E296I/M298K-FVIIA;
V158D/L287T/A294T/E296V/M298K-FVIIA;
V158D/L287T/A294T/E296L/M298K-FVIIA;      V158D/L287T/A294T/E296T/M298K-FVIIA;
V158D/L287S/A294S/E296I/M298K-FVIIA;
V158D/L287S/A294S/E296V/M298K-FVIIA;
V158D/L287S/A294S/E296L/M298K-FVIIA;      V158D/L287S/A294S/E296T/M298K-FVIIA;
V158D/L287S/A294T/E296I/M298K-FVIIA;
V158D/L287S/A294T/E296V/M298K-FVIIA;      V158D/L287S/A294T/E296L/M298K-FVIIA;
V158D/L287S/A294T/E296T/M298K-FVIIA;
V158E/L287T/A294S/E296I/M298K-FVIIA;      V158E/L287T/A294S/E296V/M298K-FVIIA;
V158E/L287T/A294S/E296L/M298K-FVIIA;
V158E/L287T/A294S/E296T/M298K-FVIIA;      V158E/L287T/A294T/E296I/M298K-FVIIA;
V158E/L287T/A294T/E296V/M298K-FVIIA;
V158E/L287T/A294T/E296L/M298K-FVIIA;      V158E/L287T/A294T/E296T/M298K-FVIIA;
V158E/L287S/A294S/E296I/M298K-FVIIA;
V158E/L287S/A294S/E296V/M298K-FVIIA;      V158E/L287S/A294S/E296L/M298K-FVIIA;
V158E/L287S/A294S/E296T/M298K-FVIIA;
V158E/L287S/A294T/E296I/M298K-FVIIA;      V158E/L287S/A294T/E296V/M298K-FVIIA;
V158E/L287S/A294T/E296L/M298K-FVIIA; and
V158E/L287S/A294T/E296T/M298K-FVIIA. In one embodiment of the invention, the Factor VII polypeptide wherein M298 has been replaced by a Lys, does not exhibit increased resistance to inactivation by an endogenous inhibitor of said FVII polypeptide relative to wild-type human FVIIa. In one embodiment of the invention, the Factor VII polypeptides, wherein M298 has been replaced by a Lys, have increased activity compared to wild-type Factor VIIa.

In a further embodiment of the invention, the Factor VII polypeptide is a polypeptide wherein at least one amino acid in the remaining positions in the protease domain has been replaced with any other amino acid.

In a further embodiment of the invention, the Factor VII polypeptide is a polypeptide wherein at the most 20 additional amino acids in the remaining positions in the protease domain have been replaced with any other amino acids.

In a further embodiment of the invention, the Factor VII polypeptide is a polypeptide wherein at least one amino acid corresponding to an amino acid at a position selected from 157-170 of SEQ ID NO:1 has been replaced with any other amino acid.

In a further embodiment of the invention, the Factor VII polypeptide is a polypeptide wherein at least one amino acid corresponding to an amino acid at a position selected from 290-305 of SEQ ID NO:1 has been replaced with any other amino acid.

In a further embodiment of the invention, the Factor VII polypeptide is a polypeptide wherein R304 has been replaced by an amino acid selected from the group consisting of Tyr, Phe, Leu, and Met.

In a further embodiment of the invention, the Factor VII polypeptide is a polypeptide wherein at least one amino acid corresponding to an amino acid at a position selected from 306-312 of SEQ ID NO:1 has been replaced with any other amino acid.

In a further embodiment of the invention, the Factor VII polypeptide is a polypeptide wherein M306 has been replaced by an amino acid selected from the group consisting of Asp, and Asn.

In a further embodiment of the invention, the Factor VII polypeptide is a polypeptide wherein D309 has been replaced by an amino acid selected from the group consisting of Ser, and Thr.

In a further embodiment of the invention, the Factor VII polypeptide is a polypeptide wherein at least one amino acid corresponding to an amino acid at a position selected from 330-339 of SEQ ID NO:1 has been replaced with any other amino acid.

In a further embodiment of the invention, the Factor VII polypeptide is a polypeptide wherein A274 has been replaced with any other amino acid.

In a further embodiment of the invention, the Factor VII polypeptide is a polypeptide wherein the A274 has been replaced by an amino acid selected from the group consisting of Met, Leu, Lys, and Arg.

In a further embodiment of the invention, the Factor VII polypeptide is a polypeptide wherein K157 has been replaced by an amino acid selected from the group consisting of Gly, Val, Ser, Thr, Asn, Gln, Asp, and Glu.

In a further embodiment of the invention, the Factor VII polypeptide is a polypeptide wherein K337 has been replaced by an amino acid selected from the group consisting of Ala, Gly, Val, Ser, Thr, Asn, Gln, Asp, and Glu.

In a further embodiment of the invention, the Factor VII polypeptide is a polypeptide wherein D334 has been replaced by an amino acid selected from the group consisting of Gly, and Glu.

In a further embodiment of the invention, the Factor VII polypeptide is a polypeptide wherein S336 has been replaced by an amino acid selected from the group consisting of Gly, and Glu.

In a further embodiment of the invention, the Factor VII polypeptide is a polypeptide wherein V158 has been replaced by an amino acid selected from the group consisting of Ser, Thr, Asn, Gln, Asp, and Glu.

In a further embodiment of the invention, the Factor VII polypeptide is a polypeptide wherein V158 has been replaced by an amino acid selected from the group consisting of Asp and Glu.

In a further embodiment of the invention, the Factor VII polypeptide is a polypeptide wherein E296 has been replaced by an amino acid selected from the group consisting of Arg, Lys, Ile, Leu, Thr and Val.

In a further embodiment of the invention, the Factor VII polypeptide is a polypeptide wherein E296 has been replaced by a Thr.

In a further embodiment of the invention, the Factor VII polypeptide is a polypeptide wherein E296 has been replaced by an amino acid selected from the group consisting of Ile, Leu, Thr, and Val.

In a further embodiment of the invention, the Factor VII polypeptide is a polypeptide wherein M298 has been replaced by an amino acid selected from the group consisting of Lys, Arg, Gln, and Asn.

In a further embodiment of the invention, the Factor VII polypeptide is a polypeptide wherein M298 has been replaced by a Lys.

In a further embodiment of the invention, the Factor VII polypeptide is a polypeptide wherein L305 has been replaced by an amino acid selected from the group consisting of Val, Tyr and Ile.

In a further embodiment of the invention, the Factor VII polypeptide is a polypeptide wherein S314 has been replaced by an amino acid selected from the group consisting of Gly, Lys, Gln and Glu.

In a further embodiment of the invention, the Factor VII polypeptide is a polypeptide wherein F374 has been replaced by an amino acid selected from the group consisting of Pro, and Tyr.

In a further embodiment of the invention, the Factor VII polypeptide is a polypeptide wherein the F374 has been replaced by Tyr.

In a further embodiment of the invention, the Factor VII polypeptide is a polypeptide wherein the amino acid has been replaced with any other amino acid which can be encoded by polynucleotide constructs.

In a further embodiment of the invention, the Factor VII polypeptide is a polypeptide wherein the Factor VII polypeptide is human Factor VII.

In a further embodiment of the invention, the Factor VII polypeptide is a polypeptide wherein the Factor VII polypeptide is human Factor VIIa.

In a further embodiment of the invention, the Factor VII polypeptide is a polypeptide wherein the ratio between the activity of the Factor VII polypeptide and the activity of the native Factor VIIa polypeptide shown in SEQ ID NO:1 is at least about 1.25.

In a further embodiment of the invention, the Factor VII polypeptide is a polypeptide wherein the ratio between the activity of the Factor VII polypeptide and the activity of the native Factor VIIa polypeptide shown in SEQ ID NO:1 is at least about 2.0, preferably at least about 4.0.

In a further embodiment of the invention, the Factor VII polypeptide is selected from Q176A-FVII, Q176L-FVII L177S-FVII, D196A-FVII, K197A-FVII, K199A-FVII, T238A-FVII, T239I-FVII, T239Y-FVII, Q286A-FVII, D289E-FVII, D289R-FVII, R290Q-FVII, M327Q-FVII, M327N-FVII, K341A-FVII, K341E-FVII, K341Q-FVII, S363M-FVII, S363A-FVII, W364H-FVII, Q366E-FVII.

In a further embodiment of the invention, the Factor VIIa polypeptides further comprises replacement of a one or more amino acids in the N-terminal Gla domain (amino acids at position corresponding to 1-37 of SEQ ID NO:1), thereby providing Factor VIIa polypeptides with a substantially higher affinity for membrane phospholipids, such as membrane phospholipids of tissue factor-bearing cells or of platelets, thereby generating Factor VII polypeptide variants which have an improved procoagulant effect.

Thus, the Factor VIIa polypeptide variants mentioned above may, in addition to the already performed amino acid replacement in positions 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 and the optional amino acid replacements to provide increased activity, also have at least one amino acid replaced in the N-terminal Gla domain, thereby obtaining a protein having an increased activity as well as an increased affinity for membrane phospholipids compared to native Factor VII. In one embodiment one or more amino acids in positions selected from 4, 8, 10, 11, 28, 32, 33, 34, and 35 (referring to SEQ ID NO: 1) of Factor VII is replaced with a different amino acid. In one embodiment one or more amino acids in positions selected from positions 10 and 32 (referring to SEQ ID NO: 1) of Factor VII is replaced with a different amino acid. Examples of preferred amino acids to be incorporated in the above-mentioned positions are: The amino acid Pro in position 10 is replaced by Gln, Arg, His, Gln, Asn or Lys; and/or the amino acid Lys in position 32 is replaced by Glu, Gln or Asn.

Other amino acids in the Gla domain, based on the different phospholipid affinities and sequences of the vitamin K-dependent plasma proteins, may also be considered for substitution.

The term "N-terminal GLA-domain" means the amino acid sequence 1-37 of Factor VII.

The three-letter indication "GLA" means 4-carboxyglutamic acid (γ-carboxyglutamate).

The term "protease domain" means the amino acid sequence 153-406 of Factor VII (the heavy-chain of Factor VIIa).

Besides in vivo clearance also functional in vivo half-life is of importance to the period of time during which the compound is "therapeutically available" in the body.

The circulating half life of recombinant human wild type FVIIa is about 2.3 hours ("Summary Basis for Approval for NovoSeven©", FDA reference number 96 0597).

The term "functional in vivo half-life" is used in its normal meaning, i.e., the time at which 50% of the biological activity of the Factor VII polypeptide or conjugate is still present in the body/target organ, or the time at which the activity of the Factor VII polypeptide is 50% of its initial value. As an alternative to determining functional in vivo half-life, "serum half-life" may be determined, i.e., the time at which 50% of the polypeptide molecules circulate in the plasma or bloodstream prior to being cleared. Determination of serum-half-life is often more simple than determining functional half-life and the magnitude of serum-half-life is usually a good indication of the magnitude of functional in vivo half-life. Alternative terms to serum half-life include plasma half-life, circulating half-life, circulatory half-life, serum clearance, plasma clearance, and clearance half-life. The polypeptide or conjugate is cleared by the action of one or more of the reticuloendothelial system (RES), kidney, spleen, or liver, by tissue factor, SEC receptor, or other receptor-mediated elimination, or by specific or unspecific proteolysis. Normally, clearance depends on size (relative to the cut-off for glomerular filtration), charge, attached carbohydrate chains, and the presence of cellular receptors for the protein. The functionality to be retained is normally selected from procoagulant, proteolytic, co-factor binding or receptor binding activity. The functional in vivo half-life and the serum half-life may be determined by any suitable method known in the art as further discussed below (see Functional Properties of Factor VII Preparations-section)

The term "increased" as used about the functional in vivo half-life or plasma half-life is used to indicate that the relevant half-life of the polypeptide or conjugate is statistically significantly increased relative to that of a reference molecule, such as wild-type human FVIIa as determined under comparable conditions. For instance the relevant half-life may be increased by at least about 25%, such as by at lest about 50%, e.g., by at least about 100%, 150%, 200%, 250%, or 500%.

As used herein, the terms "Factor VII polypeptide " or"FVII polypeptide" means any protein comprising the amino acid sequence 1-406 of wild-type human Factor VIIa (i.e., a polypeptide having the amino acid sequence disclosed in U.S. Patent No. 4,784,950), variants thereof as well as Factor VII-related polypeptides, Factor VII derivatives and Factor VII conjugates. This includes FVII variants, Factor VII-related polypeptides, Factor VII derivatives and Factor VII conjugates exhibiting substantially the same or improved biological activity relative to wild-type human Factor VIIa.

The term "Factor VII" is intended to encompass Factor VII polypeptides in their uncleaved (zymogen) form, as well as those that have been proteolytically processed to yield their respective bioactive forms, which may be designated Factor VIIa. Typically, Factor VII is cleaved between residues 152 and 153 to yield Factor VIIa. Such variants of Factor VII may exhibit different properties relative to human Factor VII, including stability, phospholipid binding, altered specific activity, and the like.

The term "factor VIIa", or "FVIIa" as used herein means a product consisting of the activated form (factor VIIa). "Factor VII" or "Factor VIIa" within the above definition also includes natural allelic variations that may exist and occur from one individual to another. Also, degree and location of glycosylation or other post-translation modifications may vary depending on the chosen host cells and the nature of the host cellular environment.

As used herein, "wild type human FVIIa" is a polypeptide having the amino acid sequence disclosed in U.S. Patent No. 4,784,950.

As used herein, "Factor VII-related polypeptides" encompasses polypeptides, including variants, in which the Factor VIIa biological activity has been substantially modified, such as reduced, relative to the activity of wild-type Factor VIIa. These polypeptides include, without limitation, Factor VII or Factor VIIa into which specific amino acid sequence alterations have been introduced that modify or disrupt the bioactivity of the polypeptide.

The term "Factor VII derivative" as used herein, is intended to designate a FVII polypeptide exhibiting substantially the same or improved biological activity relative to wild-type Factor VII, in which one or more of the amino acids of the parent peptide have been genetically and/or chemically and/or enzymatically modified, e.g. by alkylation, glycosylation, PEGylation, acylation, ester formation or amide formation or the like. This includes but is not limited to PEGylated human Factor VIIa, cysteine-PEGylated human Factor VIIa and variants thereof. Non-limiting examples of Factor VII derivatives includes GlycoPegylated FVII derivatives as disclosed in WO 03/31464 and US Patent applications US 20040043446, US 20040063911, US 20040142856, US 20040137557, and US 20040132640 (Neose Technologies, Inc.); FVII conjugates as disclosed in WO 01/04287, US patent application 20030165996, WO 01/58935, WO 03/93465 (Maxygen ApS) and WO 02/02764, US patent application 20030211094 (University of Minnesota).

The term "improved biological activity" refers to FVII polypeptides with i) substantially the same or increased proteolytic activity compared to recombinant wild type human Factor VIIa or ii) to FVII polypeptides with substantially the same or increased TF binding activity compared to recombinant wild type human Factor VIIa or iii) to FVII polypeptides with substantially the same or increased half life in blood plasma compared to recombinant wild type human Factor VIIa.The term "PEGylated human Factor VIIa" means human Factor VIIa, having a PEG molecule conjugated to a human Factor VIIa polypeptide. It is to be understood, that the PEG molecule may be attached to any part of the Factor VIIa polypeptide including any amino acid residue or carbohydrate moiety of the Factor VIIa polypeptide. The term "cysteine-PEGylated human Factor VIIa" means Factor VIIa having a PEG molecule conjugated to a sulfhydryl group of a cysteine introduced in human Factor VIIa.

Non-limiting examples of additional substitutions of amino acids in the Factor VII polypeptide providing Factor VII polypeptides with substantially the same or increased proteolytic activity compared to recombinant wild type human Factor VIIa include S52A, S60A ( Lino et al., Arch. Biochem. Biophys. 352: 182-192, 1998); substitutions providing FVIIa polypeptides exhibiting increased proteolytic stability as disclosed in U.S. Patent No. 5,580,560; substitutions providing FVIIa polypeptides that has been proteolytically cleaved between residues 290 and 291 or between residues 315 and 316 (Mollerup et al., Biotechnol. Bioeng. 48:501-505, 1995); oxidized forms of Factor VIIa (Kornfelt et al., Arch. Biochem. Biophys. 363:43-54, 1999); substitutions in FVIIa polypeptides as disclosed in WO 02/077218; and substitutions in FVIIa polypeptides, which exhibit increased proteolytic stability as disclosed in WO 02/38162 (Scripps Research Institute); substitutions in FVIIa polypeptides, which provides polypeptides having a modified Gla-domain and exhibiting an enhanced membrane binding as disclosed in WO 99/20767, US patents US 6017882 and US 6747003, US patent application 20030100506 (University of Minnesota) and WO 00/66753, US patent applications US 20010018414, US 2004220106, and US 200131005, US patents US 6762286 and US 6693075 (University of Minnesota); and substitutions in FVIIa polypeptides as disclosed in WO 01/58935, US patent US 6806063, US patent application 20030096338 (Maxygen ApS), WO 03/93465 (Maxygen ApS), WO 04/029091 (Maxygen ApS), WO 04/083361 (Maxygen ApS), and WO 04/111242 (Maxygen ApS), as well as in WO 04/108763 (Canadian Blood Services).

Non-limiting examples of further substitutions in FVIIa polypeptides providing FVII polypeptides having increased biological activity compared to wild-type FVIIa include substitutions as disclosed in WO 01/83725, WO 02/22776, WO 02/077218, WO 03/027147, WO 04/029090, WO 03/027147; WO 02/38162 (Scripps Research Institute); and FVIIa polypeptides with enhanced activity as disclosed in JP 2001061479 (Chemo-Sero-Therapeutic Res Inst.).

Examples of further substitutions in the FVIIa polypeptides of the present invention include, without limitation, L305V, L305V/M306D/D309S, L305I, L305T, F374P, V158T/M298Q, V158D/E296V/M298Q, K337A, M298Q, V158D/M298Q, L305V/K337A, V158D/E296V/M298Q/L305V, V158D/E296V/M298Q/K337A, V158D/E296V/M298Q/L305V/K337A, K157A, E296V, E296V/M298Q, V158D/E296V, V158D/M298K, and S336G, L305V/K337A, L305V/V158D, L305V/E296V, L305V/M298Q, L305V/V158T, L305V/K337A/V158T, L305V/K337A/M298Q, L305V/K337A/E296V, L305V/K337A/V158D, L305V/V158D/M298Q, L305V/V158D/E296V, L305V/V158T/M298Q, L305V/V158T/E296V, L305V/E296V/M298Q, L305V/V158D/E296V/M298Q, L305V/V158T/E296V/M298Q, L305V/V158T/K337A/M298Q, L305V/V158T/E296V/K337A, L305V/V158D/K337A/M298Q, L305V/V158D/E296V/K337A, L305V/V158D/E296V/M298Q/K337A, L305V/V158T/E296V/M298Q/K337A, S314E/K316H, S314E/K316Q, S314E/L305V, S314E/K337A, S314E/V158D, S314E/E296V, S314E/M298Q, S314E/V158T, K316H/L305V, K316H/K337A, K316H/V158D, K316H/E296V, K316H/M298Q, K316H/V158T, K316Q/L305V, K316Q/K337A, K316Q/V158D, K316Q/E296V, K316Q/M298Q, K316Q/V158T, S314E/L305V/K337A, S314E/L305V/V158D, S314E/L305V/E296V, S314E/L305V/M298Q, S314E/L305V/V158T, S314E/L305V/K337A/V158T, S314E/L305V/K337A/M298Q, S314E/L305V/K337A/E296V, S314E/L305V/K337A/V158D, S314E/L305V/V158D/M298Q, S314E/L305V/V158D/E296V, S314E/L305V/V158T/M298Q, S314E/L305V/V158T/E296V, S314E/L305V/E296V/M298Q, S314E/L305V/V158D/E296V/M298Q, S314E/L305V/V158T/E296V/M298Q, S314E/L305V/V158T/K337A/M298Q, S314E/L305V/V158T/E296V/K337A, S314E/L305V/V158D/K337A/M298Q, S314E/L305V/V158D/E296V/K337A, S314E/L305V/V158D/E296V/M298Q/K337A, S314E/L305V/V158T/E296V/M298Q/K337A, K316H/L305V/K337A, K316H/L305V/V158D, K316H/L305V/E296V, K316H/L305V/M298Q, K316H/L305V/V158T, K316H/L305V/K337A/V158T, K316H/L305V/K337A/M298Q, K316H/L305V/K337A/E296V, K316H/L305V/K337A/V158D, K316H/L305V/V158D/M298Q, K316H/L305V/V158D/E296V, K316H/L305V/V158T/M298Q, K316H/L305V/V158T/E296V, K316H/L305V/E296V/M298Q, K316H/L305V/V158D/E296V/M298Q, K316H/L305V/V158T/E296V/K337A, K316H/L305V/V158D/K337A/M298Q, K316H/L305V/V158D/E296V/K337A, K316H/L305V/V158D/E296V/M298Q/K337A, K316H/L305V/V158T/E296V/M298Q/K337A, K316Q/L305V/K337A, K316Q/L305V/V158D, K316Q/L305V/E296V, K316Q/L305V/M298Q, K316Q/L305V/V158T, K316Q/L305V/K337A/V158T, K316Q/L305V/K337A/M298Q, K316Q/L305V/K337A/E296V, K316Q/L305V/K337A/V158D, K316Q/L305V/V158D/M298Q, K316Q/L305V/V158D/E296V, K316Q/L305V/V158T/M298Q, K316Q/L305V/V158T/E296V, K316Q/L305V/E296V/M298Q, K316Q/L305V/V158D/E296V/M298Q, K316Q/L305V/V158T/E296V/M298Q, K316Q/L305V/V158T/K337A/M298Q, K316Q/L305V/V158T/E296V/K337A, K316Q/L305V/V158D/K337A/M298Q, K316Q/L305V/V158D/E296V/K337A, K316Q/L305V/V158D/E296V/M298Q/K337A, K316Q/L305V/V158T/E296V/M298Q/K337A, F374Y/K337A, F374Y/V158D, F374Y/E296V, F374Y/M298Q, F374Y/V158T, F374Y/S314E, F374Y/L305V, F374Y/ L305V/K337A, F374Y/L305V/V158D, F374Y/L305V/E296V, F374Y/L305V/M298Q, F374Y/L305V/V158T, F374Y/ L305V/S314E, F374Y/K337A/S314E, F374Y/K337A/V158T, F374Y/K337A/M298Q, F374Y/K337A/E296V, F374Y/ K337A/V158D, F374Y/V158D/S314E, F374Y/V158D/M298Q, F374Y/V158D/E296V, F374Y/V158T/S314E, F374Y/ V158T/M298Q, F374Y/V158T/E296V, F374Y/E296V/S314E, F374Y/S314E/M298Q, F374Y/E296V/M298Q, F374Y/ L305V/K337A/V158D, F374Y/L305V/K337A/E296V, F374Y/L305V/K337A/M298Q, F374Y/L305V/K337A/V158T, F374Y/L305V/K337A/S314E, F374Y/L305V/V158D/E296V, F374Y/L305V/V158D/M298Q, F374Y/L305V/V158D/ S314E, F374Y/L305V/E296V/M298Q, F374Y/L305V/E296V/V158T, F374Y/L305V/E296V/S314E, F374Y/L305V/ M298Q/V158T, F374Y/L305V/M298Q/S314E, F374Y/L305V/V158T/S314E, F374Y/K337A/S314E/V158T, F374Y/ K337A/S314E/M298Q, F374Y/K337A/S314E/E296V, F374Y/K337A/S314E/V158D, F374Y/K337A/V158T/M298Q, F374Y/K337A/V158T/E296V, F374Y/K337A/M298Q/E296V, F374Y/K337A/M298Q/V158D, F374Y/K337A/E296V/ V158D, F374Y/V158D/S314E/M298Q, F374Y/V158D/S314E/E296V, F374Y/V158D/M298Q/E296V, F374Y/V158T/ S314E/E296V, F374Y/V158T/S314E/M298Q, F374Y/V158T/M298Q/E296V, F374Y/E296V/S314E/M298Q, F374Y/ L305V/M298Q/K337A/S314E, F374Y/L305V/E296V/K337A/S314E, F374Y/E296V/M298Q/K337A/S314E, F374Y/ L305V/E296V/M298Q/K337A, F374Y/L305V/E296V/M298Q/S314E, F374Y/V158D/E296V/M298Q/K337A, F374Y/ V158D/E296V/M298Q/S314E, F374Y/L305V/V158D/K337A/S314E, F374Y/V158D/M298Q/K337A/S314E, F374Y/ V158D/E296V/K337A/S314E, F374Y/L305V/V158D/E296V/M298Q, F374Y/L305V/V158D/M298Q/K337A, F374Y/ L305V/V158D/E296V/K337A, F374Y/L305V/V158D/M298Q/S314E, F374Y/L305V/V158D/E296V/S314E, F374Y/ V158T/E296V/M298Q/K337A, F374Y/V158T/E296V/M298Q/S314E, F374Y/L305V/V158T/K337A/S314E, F374Y/ V158T/M298Q/K337A/S314E, F374Y/V158T/E296V/K337A/S314E, F374Y/L305V/V158T/E296V/M298Q, F374Y/ L305V/V158T/M298Q/K337A, F374Y/L305V/V158T/E296V/K337A, F374Y/L305V/V158T/M298Q/S314E, F374Y/ L305V/V158T/E296V/S314E, F374Y/E296V/M298Q/K337A/V158T/S314E, F374Y/V158D/E296V/M298Q/K337A/ S314E, F374Y/L305V/V158D/E296V/M298Q/S314E, F374Y/L305V/E296V/M298Q/V158T/S314E, F374Y/L305V/ E296V/M298Q/K337A/V158T, F374Y/L305V/E296V/K337A/V158T/S314E, F374Y/L305V/M298Q/K337A/V158T/ S314E, F374Y/L305V/V158D/E296V/M298Q/K337A, F374Y/L305V/V158D/E296V/K337A/S314E, F374Y/L305V/ V158D/M298Q/K337A/S314E, F374Y/L305V/E296V/M298Q/K337A/V158T/S314E, F374Y/L305V/V158D/E296V/ M298Q/K337A/S314E, S52A, S60A; R152E, S344A, T106N, K143N/N145T, V253N, R290N/A292T, G291N, R315N/V317T, K143N/N145T/R315N/V317T; and substitutions, additions or deletions in the amino acid sequence from 233Thr to 240Asn; substitutions, additions or deletions in the amino acid sequence from 304Arg to 329Cys; and substitutions, additions or deletions in the amino acid sequence from 153Ile to 223Arg.

The terms "variant" or "variants", as used herein, is intended to designate Factor VII having the sequence of SEQ ID NO: 1, wherein one or more amino acids of the parent protein have been substituted by another amino acid and/or wherein one or more amino acids of the parent protein have been deleted and/or wherein one or more amino acids have been inserted in protein and/or wherein one or more amino acids have been added to the parent protein. Such addition can take place either at the N-terminal end or at the C-terminal end of the parent protein or both. The "variant" or "variants" within this definition still have FVII activity in its activated form. In one embodiment a variant is 70 % identical with the sequence of SEQ ID NO:1. In one embodiment a variant is 80 % identical with the sequence of SEQ ID NO:1. In another embodiment a variant is 90 % identical with the sequence of SEQ ID NO:1. In a further embodiment a variant is 95 % identical with the sequence of SEQ ID NO:1.

The term "construct" is intended to indicate a polynucleotide segment which may be based on a complete or partial naturally occurring nucleotide sequence encoding the polypeptide of interest. The construct may optionally contain other polynucleotide segments. In a similar way, the term "amino acids which can be encoded by polynucleotide constructs" covers amino acids which can be encoded by the polynucleotide constructs defined above, i.e. amino acids such as Ala, Val, Leu, Ile, Met, Phe, Trp, Pro, Gly, Ser, Thr, Cys, Tyr, Asn, Glu, Lys, Arg, His, Asp and Gln.

The term "vector", as used herein, means any nucleic acid entity capable of the amplification in a host cell. Thus, the vector may be an autonomously replicating vector, i.e. a vector, which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated. The choice of vector will often depend on the host cell into which it is to be introduced. Vectors include, but are not limited to plasmid vectors, phage vectors, viruses or cosmid vectors. Vectors usually contains a replication origin and at least one selectable gene, i.e., a gene which encodes a product which is readily detectable or the presence of which is essential for cell growth.

In a further aspect, the invention provides a recombinant host cell comprising the polynucleotide construct or the vector. In one embodiment the recombinant host cell is a eukaryotic cell. In another embodiment the recombinant host cell is of mammalian origin. In a further embodiment the recombinant host cell is selected from the group consisting of CHO cells , HEK cells and BHK cells.

The term "a host cell", as used herein, represent any cell, including hybrid cells, in which heterologous DNA can be expressed. Typical host cells includes, but are not limited to insect cells, yeast cells, mammalian cells, including human cells, such as BHK, CHO, HEK, and COS cells. In practicing the present invention, the host cells being cultivated are preferably mammalian cells, more preferably an established mammalian cell line, including, without limitation, CHO (e.g., ATCC CCL 61), COS-1 (e.g., ATCC CRL 1650), baby hamster kidney (BHK) and HEK293 (e.g., ATCC CRL 1573; Graham et al., J. Gen. Virol. 36:59-72, 1977) cell lines. A preferred BHK cell line is the tk- ts13 BHK cell line (Waechter and Baserga, Proc.Natl.Acad.Sci.USA 79:1106-1110, 1982), hereinafter referred to as BHK 570 cells. The BHK 570 cell line is available from the American Type Culture Collection, 12301 Parklawn Dr., Rockville, MD 20852, under ATCC accession number CRL 10314. A tk- ts13 BHK cell line is also available from the ATCC under accession number CRL 1632. Other suitable cell lines include, without limitation, Rat Hep I (Rat hepatoma; ATCC CRL 1600), Rat Hep II (Rat hepatoma; ATCC CRL 1548), TCMK (ATCC CCL 139), Human lung (ATCC HB 8065), NCTC 1469 (ATCC CCL 9.1) and DUKX cells (Urlaub and Chasin, Proc. Natl. Acad. Sci. USA 77:4216-4220, 1980). Also useful are 3T3 cells, Namalwa cells, myelomas and fusions of myelomas with other cells.

In a further aspect, the invention provides a transgenic animal containing and expressing the polynucleotide construct.

In a further aspect, the invention provides a transgenic plant containing and expressing the polynucleotide construct.

In a further aspect, the invention relates to a method for producing the Factor VII polypeptide of the invention, the method comprising cultivating a cell comprising the polynucleotide construct in an appropriate growth medium under conditions allowing expression of the polynucleotide construct and recovering the resulting polypeptide from the culture medium.

As used herein the term "appropriate growth medium" means a medium containing nutrients and other components required for the growth of cells and the expression of the nucleic acid sequence encoding the Factor VII polypeptide of the invention.

In a further aspect, the invention relates to a method for producing the Factor VII polypeptide, the method comprising recovering the polypeptide from milk produced by the transgenic animal.

In a further aspect, the invention relates to a method for producing the Factor VII polypeptide, the method comprising cultivating a cell of a transgenic plant comprising the polynucleotide construct, and recovering the polypeptide from the resulting plant.

In a further aspect, the invention relates to a pharmaceutical composition comprising a Factor VII polypeptide comprising one or more substitutions relative to the amino acid sequence of SEQ ID NO: 1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide has increased functional in vivo half-life as compared to human wild-type Factor VIIa; and, optionally, a pharmaceutically acceptable carrier.

In a further aspect, the invention relates to the use of a Factor VII polypeptide comprising one or more substitutions relative to the amino acid sequence of SEQ ID NO: 1, wherein the substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein the Factor VII polypeptide has increased functional in vivo half-life as compared to human wild-type Factor VIIa; for the preparation of a medicament for the treatment of bleeding disorders or bleeding episodes or for the enhancement of the normal haemostatic system. In one embodiment the use is for the treatment of haemophilia A or B.

In the present context, the term "treatment" is meant to include both prevention of an expected bleeding, such as in surgery, and regulation of an already occurring bleeding, such as in trauma, with the purpose of inhibiting or minimising the bleeding. Prophylactic administration of the Factor VIIa polypeptide according to the invention is thus included in the term "treatment".

The term "bleeding episodes" is meant to include uncontrolled and excessive bleeding. Bleeding episodes may be a major problem both in connection with surgery and other forms of tissue damage. Uncontrolled and excessive bleeding may occur in subjects having a normal coagulation system and subjects having coagulation or bleeding disorders. As used herein the term "bleeding disorder" reflects any defect, congenital, acquired or induced, of cellular or molecular origin that is manifested in bleedings. Examples are clotting factor deficiencies (e.g. haemophilia A and B or deficiency of coagulation Factors XI or VII), clotting factor inhibitors, defective platelet function, thrombocytopenia or von Willebrand's disease.

Excessive bleedings also occur in subjects with a normally functioning blood clotting cascade (no clotting factor deficiencies or -inhibitors against any of the coagulation factors) and may be caused by a defective platelet function, thrombocytopenia or von Willebrand's disease. In such cases, the bleedings may be likened to those bleedings caused by haemophilia because the haemostatic system, as in haemophilia, lacks or has abnormal essential clotting "compounds" (such as platelets or von Willebrand factor protein) that causes major bleedings. In subjects who experience extensive tissue damage in association with surgery or vast trauma, the normal haemostatic mechanism may be overwhelmed by the demand of immediate haemostasis and they may develop bleeding in spite of a normal haemostatic mechanism. Achieving satisfactory haemostasis also is a problem when bleedings occur in organs such as the brain, inner ear region and eyes with limited possibility for surgical haemostasis. The same problem may arise in the process of taking biopsies from various organs (liver, lung, tumour tissue, gastrointestinal tract) as well as in laparoscopic surgery. Common for all these situations is the difficulty to provide haemostasis by surgical techniques (sutures, clips, etc.) which also is the case when bleeding is diffuse (haemorrhagic gastritis and profuse uterine bleeding). Acute and profuse bleedings may also occur in subjects on anticoagulant therapy in whom a defective haemostasis has been induced by the therapy given. Such subjects may need surgical interventions in case the anticoagulant effect has to be counteracted rapidly. Radical retropubic prostatectomy is a commonly performed procedure for subjects with localized prostate cancer. The operation is frequently complicated by significant and sometimes massive blood loss. The considerable blood loss during prostatectomy is mainly related to the complicated anatomical situation, with various densely vascularized sites that are not easily accessible for surgical haemostasis, and which may result in diffuse bleeding from a large area. Another situation that may cause problems in the case of unsatisfactory haemostasis is when subjects with a normal haemostatic mechanism are given anticoagulant therapy to prevent thromboembolic disease. Such therapy may include heparin, other forms of proteoglycans, warfarin or other forms of vitamin K-antagonists as well as aspirin and other platelet aggregation inhibitors.

In one embodiment of the invention, the bleeding is associated with haemophilia. In another embodiment, the bleeding is associated with haemophilia with aquired inhibitors. In another embodiment, the bleeding is associated with thrombocytopenia. In another embodiment, the bleeding is associated with von Willebrand's disease. In another embodiment, the bleeding is associated with severe tissue damage. In another embodiment, the bleeding is associated with severe trauma. In another embodiment, the bleeding is associated with surgery. In another embodiment, the bleeding is associated with laparoscopic surgery. In another embodiment, the bleeding is associated with haemorrhagic gastritis. In another embodiment, the bleeding is profuse uterine bleeding. In another embodiment, the bleeding is occurring in organs with a limited possibility for mechanical haemostasis. In another embodiment, the bleeding is occurring in the brain, inner ear region or eyes. In another embodiment, the bleeding is associated with the process of taking biopsies. In another embodiment, the bleeding is associated with anticoagulant therapy.

The term "subject" as used herein is intended to mean any animal, in particular mammals, such as humans, and may, where appropriate, be used interchangeably with the term "patient".

The term "enhancement of the normal haemostatic system" means an enhancement of the ability to generate thrombin.

In a further aspect, the invention relates to a method for the treatment of bleeding disorders or bleeding episodes in a subject or for the enhancement of the normal haemostatic system, the method comprising administering a therapeutically or prophylactically effective amount of a Factor VII polypeptide ............; to a subject in need thereof.

In a further aspect, the invention relates to the Factor VII polypeptide of the invention for use as a medicament.

In one embodiment of the invention, the factor VII polypeptide is a polypeptide, wherein A175 is replaced with any other amino acid.

In one embodiment of the invention, the factor VII polypeptide is a polypeptide, wherein Q176 is replaced with any other amino acid.

In one embodiment of the invention, the factor VII polypeptide is a polypeptide, wherein L177 is replaced with any other amino acid.

In one embodiment of the invention, the factor VII polypeptide is a polypeptide, wherein D196 is re-placed with any other amino acid.

In one embodiment of the invention, the factor VII polypeptide is a polypeptide, wherein K197 is replaced with any other amino acid.

In one embodiment of the invention, the factor VII polypeptide is a polypeptide, wherein I198 is replaced with any other amino acid.

In one embodiment of the invention, the factor VII polypeptide is a polypeptide, wherein K199 is replaced with any other amino acid.

In one embodiment of the invention, the factor VII polypeptide is a polypeptide, wherein G237 is replaced with any other amino acid.

In one embodiment of the invention, the factor VII polypeptide is a polypeptide, wherein T238 is replaced with any other amino acid.

In one embodiment of the invention, the factor VII polypeptide is a polypeptide, wherein T239 is replaced with any other amino acid.

In one embodiment of the invention, the factor VII polypeptide is a polypeptide, wherein Q286 is replaced with any other amino acid.

In one embodiment of the invention, the factor VII polypeptide is a polypeptide, wherein L287 is replaced with any other amino acid.

In one embodiment of the invention, the factor VII polypeptide is a polypeptide, wherein L288 is replaced with any other amino acid.

In one embodiment of the invention, the factor VII polypeptide is a polypeptide, wherein D289 is replaced with any other amino acid.

In one embodiment of the invention, the factor VII polypeptide is a polypeptide, wherein R290 is replaced with any other amino acid.

In one embodiment of the invention, the factor VII polypeptide is a polypeptide, wherein G291 is replaced with any other amino acid.

In one embodiment of the invention, the factor VII polypeptide is a polypeptide, wherein A292 is replaced with any other amino acid.

In one embodiment of the invention, the factor VII polypeptide is a polypeptide, wherein T293 is replaced with any other amino acid.

In one embodiment of the invention, the factor VII polypeptide is a polypeptide, wherein A294 is replaced with any other amino acid.

In one embodiment of the invention, the factor VII polypeptide is a polypeptide, wherein L295 is replaced with any other amino acid.

In one embodiment of the invention, the factor VII polypeptide is a polypeptide, wherein L297 is replaced with any other amino acid.

In one embodiment of the invention, the factor VII polypeptide is a polypeptide, wherein V299 is replaced with any other amino acid.

In one embodiment of the invention, the factor VII polypeptide is a polypeptide, wherein M327 is replaced with any other amino acid.

In one embodiment of the invention, the factor VII polypeptide is a polypeptide, wherein K341 is replaced with any other amino acid.

In one embodiment of the invention, the factor VII polypeptide is a polypeptide, wherein S363 is replaced with any other amino acid.

In one embodiment of the invention, the factor VII polypeptide is a polypeptide, wherein W364 is replaced with any other amino acid.

In one embodiment of the invention, the factor VII polypeptide is a polypeptide, wherein G365 is replaced with any other amino acid.

In one embodiment of the invention, the factor VII polypeptide is a polypeptide, wherein Q366 is replaced with any other amino acid.

In one embodiment of the invention, the factor VII polypeptide is a polypeptide, wherein T239 is replaced with any other amino acid selected from Ile and Tyr.

In one embodiment of the invention, the factor VII polypeptide is a polypeptide, wherein D289 is replaced with any other amino acid selected from Glu and Arg.

In one embodiment of the invention, the factor VII polypeptide is a polypeptide, wherein M327 is replaced with any other amino acid selected from Gln and Asn.

In one embodiment of the invention, the factor VII polypeptide is a polypeptide, wherein K341 is replaced with any other amino acid selected from Ala, Glu, and Gln.

In one embodiment of the invention, the factor VII polypeptide is a polypeptide, wherein S363 is replaced with any other amino acid selected from Met and Ala.

In one embodiment of the invention, the factor VII polypeptide is a polypeptide, wherein at least one amino acid in the remaining positions in the protease domain has been re-placed with any other amino acid.

In one embodiment of the invention, the factor VII polypeptide is a polypeptide, wherein at the most 20 additional amino acids in the remaining positions in the protease domain have been replaced with any other amino acids.

In one embodiment of the invention, the factor VII polypeptide is a polypeptide, wherein at least one amino acid corresponding to an amino acid at a position selected from 157-170 of SEQ ID NO:1 has been replaced with any other amino acid.

In one embodiment of the invention, the factor VII polypeptide is a polypeptide, wherein at least one amino acid corresponding to an amino acid at a position selected from 290-305 of SEQ ID NO:1 has been replaced with any other amino acid.

In one embodiment of the invention, the factor VII polypeptide is a polypeptide, wherein R304 has been replaced by an amino acid selected from the group consisting of Tyr, Phe, Leu, and Met.

In one embodiment of the invention, the factor VII polypeptide is a polypeptide, wherein at least one amino acid corresponding to an amino acid at a position selected from 306-312 of SEQ ID NO:1 has been replaced with any other amino acid.

In one embodiment of the invention, the factor VII polypeptide is a polypeptide, wherein M306 has been replaced by an amino acid selected from the group consisting of Asp, and Asn.

In one embodiment of the invention, the factor VII polypeptide is a polypeptide, wherein D309 has been replaced by an amino acid selected from the group consisting of Ser, and Thr.

In one embodiment of the invention, the factor VII polypeptide is a polypeptide, wherein at least one amino acid corresponding to an amino acid at a position selected from 330-339 of SEQ ID NO:1 has been replaced with any other amino acid.

In one embodiment of the invention, the factor VII polypeptide is a polypeptide, wherein A274 has been replaced with any other amino acid.

In one embodiment of the invention, the factor VII polypeptide is a polypeptide, wherein the A274 has been replaced by an amino acid selected from the group consisting of Met, Leu, Lys, and Arg.

In one embodiment of the invention, the factor VII polypeptide is a polypeptide, wherein K157 has been replaced by an amino acid selected from the group consisting of Gly, Val, Ser, Thr, Asn, Gln, Asp, and Glu.

In one embodiment of the invention, the factor VII polypeptide is a polypeptide, wherein K337 has been replaced by an amino acid selected from the group consisting of Ala, Gly, Val, Ser, Thr, Asn, Gln, Asp, and Glu.

In one embodiment of the invention, the factor VII polypeptide is a polypeptide, wherein D334 has been replaced by an amino acid selected from the group consisting of Gly, and Glu.

In one embodiment of the invention, the factor VII polypeptide is a polypeptide, wherein S336 has been replaced by an amino acid selected from the group consisting of Gly, and Glu.

In one embodiment of the invention, the factor VII polypeptide is a polypeptide, wherein V158 has been replaced by an amino acid selected from the group consisting of Ser, Thr, Asn, Gln, Asp, and Glu.

In one embodiment of the invention, the factor VII polypeptide is a polypeptide, wherein E296 has been replaced by an amino acid selected from the group consisting of Arg, Lys, Ile, Leu and Val.

In one embodiment of the invention, the factor VII polypeptide is a polypeptide, wherein M298 has been replaced by an amino acid selected from the group consisting of Lys, Arg, Gln, and Asn.

In one embodiment of the invention, the factor VII polypeptide is a polypeptide, wherein L305 has been replaced by an amino acid selected from the group consisting of Val, Tyr and Ile.

In one embodiment of the invention, the factor VII polypeptide is a polypeptide, wherein S314 has been replaced by an amino acid selected from the group consisting of Gly, Lys, Gln and Glu.

In one embodiment of the invention, the factor VII polypeptide is a polypeptide, wherein F374 has been replaced by an amino acid selected from the group consisting of Pro, and Tyr.

In one embodiment of the invention, the factor VII polypeptide is a polypeptide, wherein the F374 has been replaced by Tyr.

In one embodiment of the invention, the factor VII polypeptide is a polypeptide, wherein the amino acid has been replaced with any other amino acid which can be encoded by polynucleotide constructs.

In one embodiment of the invention, the factor VII polypeptide is a polypeptide, wherein the Factor VII polypeptide is human Factor VII.

In one embodiment of the invention, the factor VII polypeptide is a polypeptide, wherein the Fac-tor VII polypeptide is human Factor VIIa.

In one embodiment of the invention, the factor VII polypeptide is a polypeptide, wherein the ratio between the activity of the Factor VII polypeptide and the activity of the native Factor VIIa polypeptide shown in SEQ ID NO:1 is at least about 1.25.

In one embodiment of the invention, the factor VII polypeptide is a polypeptide, wherein the ratio is at least about 2.0, preferably at least about 4.0.

In one embodiment of the invention, the factor VII polypeptide is a polypeptide, which is selected from Q176A-FVII, Q176L-FVII L177S-FVII, D196A-FVII, K197A-FVII, K199A-FVII, T238A-FVII, T2391-FVII, T239Y-FVII, Q286A-FVII, D289E-FVII, D289R-FVII, R290Q-FVII, M327Q-FVII, M327N-FVII, K341A-FVII, K341E-FVII, K341Q-FVII, S363M-FVII, S363A-FVII, W364H-FVII, Q366E-FVII.

In a further embodiment of the invention, the factor VII polypeptide is a polypeptide, wherein at least one amino acid in the remaining positions in the protease domain has been replaced with any other amino acid.

In a further embodiment of the invention, the factor VII polypeptide is a polypeptide, wherein at the most 20 additional amino acids in the remaining positions in the protease domain have been replaced with any other amino acids.

In a further embodiment of the invention, the factor VII polypeptide is a polypeptide, wherein the amino acid has been replaced by a different amino acid which can be encoded by polynucleotide constructs.

In a further embodiment of the invention, the factor VII polypeptide is a polypeptide, wherein the Factor VII polypeptide is human Factor VII.

In a further embodiment of the invention, the factor VII polypeptide is a polypeptide, wherein the Factor VII polypeptide is human Factor VIIa.

In a further embodiment of the invention, the factor VII polypeptide is a polypeptide, wherein the ratio between the activity of the Factor VII polypeptide and the activity of the native Factor VIIa polypeptide shown in SEQ ID NO:1 is at least about 1.25. In one embodiment the ratio between the activity of the Factor VII polypeptide and the activity of the native Factor VIIa polypeptide shown in SEQ ID NO:1 is at least about 2.0. In a further embodiment the ratio between the activity of the Factor VII polypeptide and the activity of the native Factor VIIa polypeptide shown in SEQ ID NO:1 is at least about 4.0.

In a further embodiment of the invention, the factor VII polypeptide is a polypeptide, wherein the ratio between the activity of the Factor VII polypeptide and the activity of the native Factor VIIa polypeptide shown in SEQ ID NO:1 is at least about 1.25 when tested in a Factor VIIa activity assay. In one embodiment the ratio between the activity of the Factor VII polypeptide and the activity of the native Factor VIIa polypeptide shown in SEQ ID NO:1 is at least about 2.0 when tested in a Factor VIIa activity assay. In a further embodiment the ratio between the activity of the Factor VII polypeptide and the activity of the native Factor VIIa polypeptide shown in SEQ ID NO:1 is at least about 4.0 when tested in a Factor VIIa activity assay. The Factor VIIa activity may be measured by the assays described in examples 4 or 5.

In a further embodiment of the invention, the factor VII polypeptide is a polypeptide, wherein the ratio between the activity of the Factor VII polypeptide and the activity of the native Factor VIIa polypeptide shown in SEQ ID NO:1 is at least about 1.25 when tested in the *"In Vitro* Hydrolysis Assay". In one embodiment the ratio between the activity of the Factor VII polypeptide and the activity of the native Factor VIIa polypeptide shown in SEQ ID NO:1 is at least about 2.0 when tested in the *"In Vitro* Hydrolysis Assay". In a further embodiment the ratio between the activity of the Factor VII polypeptide and the activity of the native Factor VIIa polypeptide shown in SEQ ID NO:1 is at least about 4.0 when tested in the *"In Vitro* Hydrolysis Assay".

In a further embodiment of the invention, the factor VII polypeptide is a polypeptide, wherein the ratio between the activity of the Factor VII polypeptide and the activity of the native Factor VIIa polypeptide shown in SEQ ID NO:1 is at least about 1.25 when tested in the *"In Vitro* Proteolysis Assay". In one embodiment the ratio between the activity of the Factor VII polypeptide and the activity of the native Factor VIIa polypeptide shown in SEQ ID NO:1 is at least about 2.0 when tested in the *"In Vitro* Proteolysis Assay". In a further embodiment the ratio between the activity of the Factor VII polypeptide and the activity of the native Factor VIIa polypeptide shown in SEQ ID NO:1 is at least about 4.0 when tested in the *"In Vitro* Proteolysis Assay". In a further embodiment the ratio between the activity of the Factor VII polypeptide and the activity of the native Factor VIIa polypeptide shown in SEQ ID NO:1 is at least about 8.0 when tested in the *"In Vitro* Proteolysis Assay".

In a further aspect, the invention provides human coagulation Factor VIIa polypeptides that have increased tissue factor-independent activity compared to native human coagulation Factor VIIa. In another aspect, the increased activity is not accompanied by changes in the substrate specificity. In another aspect of the invention, the binding of the polypeptide variants to tissue factor should not be impaired and the polypeptide variants should have at least the activity of wild-type Factor VIIa when bound to tissue factor.

The terminology for amino acid substitutions used in this description are as follows. The first letter represent the amino acid naturally present at a position of SEQ ID NO:1. The following number represent the position in SEQ ID NO:1. The second letter represent the different amino acid substituting for the natural amino acid. An example is K197A-FVII, wherein the Lysine at position 197 of SEQ ID NO:1 is replaced by a Alanine.

In the present context the three-letter or one-letter indications of the amino acids have been used in their conventional meaning as indicated in table 1. Unless indicated explicitly, the amino acids mentioned herein are L-amino acids. Further, the left and right ends of an amino acid sequence of a peptide are, respectively, the N- and C-termini unless otherwise specified.

**Table 1: Abbreviations for amino acids:**

| Amino acid | Tree-letter code | One-letter code |
|---|---|---|
| Glycine | Gly | G |
| Proline | Pro | P |
| Alanine | Ala | A |
| Valine | Val | V |

(continued)

| Amino acid | Tree-letter code | One-letter code |
|---|---|---|
| Leucine | Leu | L |
| Isoleucine | Ile | I |
| Methionine | Met | M |
| Cysteine | Cys | C |
| Phenylalanine | Phe | F |
| Tyrosine | Tyr | Y |
| Tryptophan | Trp | W |
| Histidine | His | H |
| Lysine | Lys | K |
| Arginine | Arg | R |
| Glutamine | Gln | Q |
| Asparagine | Asn | N |
| Glutamic Acid | Glu | E |
| Aspartic Acid | Asp | D |
| Serine | Ser | S |
| Threonine | Thr | T |

Preparation of Factor VII polypeptide variants

The invention also relates to a method of preparing human Factor VII polypeptide variants as mentioned above. The Factor VII polypeptide variants described herein may be produced by means of recombinant nucleic acid techniques. In general, a cloned wild-type Factor VII nucleic acid sequence is modified to encode the desired protein. This modified sequence is then inserted into an expression vector, which is in turn transformed or transfected into host cells. Higher eukaryotic cells, in particular cultured mammalian cells, are preferred as host cells. The complete nucleotide and amino acid sequences for human Factor VII are known (see U.S. 4,784,950, where the cloning and expression of recombinant human Factor VII is described). The bovine Factor VII sequence is described in Takeya et al., J. Biol. Chem. 263: 14868-14872 (1988)).

The amino acid sequence alterations may be accomplished by a variety of techniques. Modification of the nucleic acid sequence may be by site-specific mutagenesis. Techniques for site-specific mutagenesis are well known in the art and are described in, for example, Zoller and Smith (DNA 3:479-488, 1984) or "Splicing by extension overlap", Horton et al., Gene 77, 1989, pp. 61-68. Thus, using the nucleotide and amino acid sequences of Factor VII, one may introduce the alteration(s) of choice. Likewise, procedures for preparing a DNA construct using polymerase chain reaction using specific primers are well known to persons skilled in the art (cf. PCR Protocols, 1990, Academic Press, San Diego, California, USA).

The nucleic acid construct encoding the Factor VII polypeptide variant of the invention may suitably be of genomic or cDNA origin, for instance obtained by preparing a genomic or cDNA library and screening for DNA sequences coding for all or part of the polypeptide by hybridization using synthetic oligonucleotide probes in accordance with standard techniques (cf. Sambrook et al., Molecular Cloning: A Laboratory Manual, 2nd. Ed. Cold Spring Harbor Laboratory, Cold Spring Harbor, New York, 1989).

The nucleic acid construct encoding the Factor VII polypeptide variant may also be prepared synthetically by established standard methods, e.g. the phosphoamidite method described by Beaucage and Caruthers, Tetrahedron Letters 22 (1981), 1859 - 1869, or the method described by Matthes et al., EMBO Journal 3 (1984), 801 - 805. According to the phosphoamidite method, oligonucleotides are synthesised, e.g. in an automatic DNA synthesiser, purified, annealed, ligated and cloned in suitable vectors. The DNA sequences encoding the human Factor VII polypeptide variants may also be prepared by polymerase chain reaction using specific primers, for instance as described in US 4,683,202, Saiki et al., Science 239 (1988), 487 - 491, or Sambrook et al., *supra.*

Furthermore, the nucleic acid construct may be of mixed synthetic and genomic, mixed synthetic and cDNA or mixed genomic and cDNA origin prepared by ligating fragments of synthetic, genomic or cDNA origin (as appropriate), the fragments corresponding to various parts of the entire nucleic acid construct, in accordance with standard techniques.

The nucleic acid construct is preferably a DNA construct. DNA sequences for use in producing Factor VII polypeptide variants according to the present invention will typically encode a pre-pro polypeptide at the amino-terminus of Factor VII to obtain proper posttranslational processing (e.g. gamma-carboxylation of glutamic acid residues) and secretion from the host cell. The pre-pro polypeptide may be that of Factor VII or another vitamin K-dependent plasma protein, such as Factor IX, Factor X, prothrombin, protein C or protein S. As will be appreciated by those skilled in the art, additional modifications can be made in the amino acid sequence of the Factor VII polypeptide variants where those modifications do not significantly impair the ability of the protein to act as a coagulant. For example, the Factor VII polypeptide variants can also be modified in the activation cleavage site to inhibit the conversion of zymogen Factor VII into its activated two-chain form, as generally described in U.S. 5,288,629, incorporated herein by reference.

The DNA sequences encoding the human Factor VII polypeptide variants are usually inserted into a recombinant vector which may be any vector, which may conveniently be subjected to recombinant DNA procedures, and the choice of vector will often depend on the host cell into which it is to be introduced. Thus, the vector may be an autonomously replicating vector, i.e. a vector, which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

The vector is preferably an expression vector in which the DNA sequence encoding the human Factor VII polypeptide variants is operably linked to additional segments required for transcription of the DNA. In general, the expression vector is derived from plasmid or viral DNA, or may contain elements of both. The term, "operable linked" indicates that the segments are arranged so that they function in concert for their intended purposes, e.g. transcription initiates in a promoter and proceeds through the DNA sequence coding for the polypeptide.

Expression vectors for use in expressing Factor VIIa polypeptide variants will comprise a promoter capable of directing the transcription of a cloned gene or cDNA. The promoter may be any DNA sequence, which shows transcriptional activity in the host cell of choice and may be derived from genes encoding proteins either homologous or heterologous to the host cell.

Examples of suitable promoters for directing the transcription of the DNA encoding the human Factor VII polypeptide variant in mammalian cells are the SV40 promoter (Subramani et al., Mol. Cell Biol. 1 (1981), 854 -864), the MT-1 (metallothionein gene) promoter (Palmiter et al., Science 222 (1983), 809 - 814), the CMV promoter (Boshart et al., Cell 41:521-530, 1985) or the adenovirus 2 major late promoter (Kaufman and Sharp, Mol. Cell. Biol, 2:1304-1319, 1982).

An example of a suitable promoter for use in insect cells is the polyhedrin promoter (US 4,745,051; Vasuvedan et al., FEBS Lett. 311, (1992) 7 - 11), the P10 promoter (J.M. Vlak et al., J. Gen. Virology 69, 1988, pp. 765-776), the *Autographa californica* polyhedrosis virus basic protein promoter (EP 397 485), the baculovirus immediate early gene 1 promoter (US 5,155,037; US 5,162,222), or the baculovirus 39K delayed-early gene promoter (US 5,155,037; US 5,162,222).

Examples of suitable promoters for use in yeast host cells include promoters from yeast glycolytic genes (Hitzeman et al., J. Biol. Chem. 255 (1980), 12073 - 12080; Alber and Kawasaki, J. Mol. Appl. Gen. 1 (1982), 419 - 434) or alcohol dehydrogenase genes (Young et al., in Genetic Engineering of Microorganisms for Chemicals (Hollaender et al, eds.), Plenum Press, New York, 1982), or the TPI1 (US 4,599,311) or ADH2-4c (Russell et al., Nature 304 (1983), 652 - 654) promoters.

Examples of suitable promoters for use in filamentous fungus host cells are, for instance, the ADH3 promoter (McKnight et al., The EMBO J. 4 (1985), 2093 - 2099) or the tpiA promoter. Examples of other useful promoters are those derived from the gene encoding *A. oryzae* TAKA amylase, *Rhizomucor miehei* aspartic proteinase, *A. niger* neutral α-amylase, *A. niger* acid stable α-amylase, *A. niger* or *A. awamori* glucoamylase (gluA), *Rhizomucor miehei* lipase, *A. oryzae* alkaline protease, *A. oryzae* triose phosphate isomerase or *A. nidulans* acetamidase. Preferred are the TAKA-amylase and gluA promoters. Suitable promoters are mentioned in, e.g. EP 238 023 and EP 383 779.

The DNA sequences encoding the human Factor VII polypeptide variants may also, if necessary, be operably connected to a suitable terminator, such as the human growth hormone terminator (Palmiter et al., Science 222, 1983, pp. 809-814) or the TPI1 (Alber and Kawasaki, J. Mol. Appl. Gen. 1, 1982, pp. 419-434) or ADH3 (McKnight et al., The EMBO J. 4, 1985, pp. 2093-2099) terminators. Expression vectors may also contain a set of RNA splice sites located downstream from the promoter and upstream from the insertion site for the Factor VII sequence itself. Preferred RNA splice sites may be obtained from adenovirus and/or immunoglobulin genes. Also contained in the expression vectors is a polyadenylation signal located downstream of the insertion site. Particularly preferred polyadenylation signals include the early or late polyadenylation signal from SV40 (Kaufman and Sharp, ibid.), the polyadenylation signal from the adenovirus 5 Elb region, the human growth hormone gene terminator (DeNoto et al. Nucl. Acids Res. 9:3719-3730, 1981) or the polyadenylation signal from the human Factor VII gene or the bovine Factor VII gene. The expression vectors may also include a noncoding viral leader sequence, such as the adenovirus 2 tripartite leader, located between the promoter and the RNA splice sites; and enhancer sequences, such as the SV40 enhancer.

To direct the human Factor VII polypeptide variants of the present invention into the secretory pathway of the host cells, a secretory signal sequence (also known as a leader sequence, prepro sequence or pre sequence) may be provided in the recombinant vector. The secretory signal sequence is joined to the DNA sequences encoding the human Factor VII polypeptide variants in the correct reading frame. Secretory signal sequences are commonly positioned 5' to the DNA sequence encoding the peptide. The secretory signal sequence may be that, normally associated with the protein or may be from a gene encoding another secreted protein.

For secretion from yeast cells, the secretory signal sequence may encode any signal peptide, which ensures efficient direction of the expressed human Factor VII polypeptide variants into the secretory pathway of the cell. The signal peptide may be naturally occurring signal peptide, or a functional part thereof, or it may be a synthetic peptide. Suitable signal peptides have been found to be the α-factor signal peptide (cf. US 4,870,008), the signal peptide of mouse salivary amylase (cf. O. Hagenbuchle et al., Nature 289, 1981, pp. 643-646), a modified carboxypeptidase signal peptide (cf. L.A. Valls et al., Cell 48, 1987, pp. 887-897), the yeast BAR1 signal peptide (cf. WO 87/02670), or the yeast aspartic protease 3 (YAP3) signal peptide (cf. M. Egel-Mitani et al., Yeast 6, 1990, pp. 127-137).

For efficient secretion in yeast, a sequence encoding a leader peptide may also be inserted downstream of the signal sequence and upstream of the DNA sequence encoding the human Factor VII polypeptide variants. The function of the leader peptide is to allow the expressed peptide to be directed from the endoplasmic reticulum to the Golgi apparatus and further to a secretory vesicle for secretion into the culture medium (i.e. exportation of the human Factor VII polypeptide variants across the cell wall or at least through the cellular membrane into the periplasmic space of the yeast cell). The leader peptide may be the yeast alpha-factor leader (the use of which is described in e.g. US 4,546,082, US 4,870,008, EP 16 201, EP 123 294, EP 123 544 and EP 163 529). Alternatively, the leader peptide may be a synthetic leader peptide, which is to say a leader peptide not found in nature. Synthetic leader peptides may, for instance, be constructed as described in WO 89/02463 or WO 92/11378.

For use in filamentous fungi, the signal peptide may conveniently be derived from a gene encoding an *Aspergillus* sp. amylase or glucoamylase, a gene encoding a *Rhizomucor miehei* lipase or protease or a *Humicola lanuginosa* lipase. The signal peptide is preferably derived from a gene encoding *A. oryzae* TAKA amylase, *A. niger* neutral α-amylase, *A. niger* acid-stable amylase, or *A. niger* glucoamylase. Suitable signal peptides are disclosed in, e.g. EP 238 023 and EP 215 594.

For use in insect cells, the signal peptide may conveniently be derived from an insect gene (cf. WO 90/05783), such as the lepidopteran *Manduca sexta* adipokinetic hormone precursor signal peptide (cf. US 5,023,328).

The procedures used to ligate the DNA sequences coding for the human Factor VII polypeptide variants, the promoter and optionally the terminator and/or secretory signal sequence, respectively, and to insert them into suitable vectors containing the information necessary for replication, are well known to persons skilled in the art (cf., for instance, Sambrook et al., Molecular Cloning: A Laboratory Manual, Cold Spring Harbor, New York, 1989).

Methods of transfecting mammalian cells and expressing DNA sequences introduced in the cells are described in e.g. Kaufman and Sharp, J. Mol. Biol. 159 (1982), 601 - 621; Southern and Berg, J. Mol. Appl. Genet. 1 (1982), 327 - 341; Loyter et al., Proc. Natl. Acad. Sci. USA 79 (1982), 422 - 426; Wigler et al., Cell 14 (1978), 725; Corsaro and Pearson, Somatic Cell Genetics 7 (1981), 603, Graham and van der Eb, Virology 52 (1973), 456; and Neumann et al., EMBO J. 1 (1982), 841 - 845.

Cloned DNA sequences are introduced into cultured mammalian cells by, for example, calcium phosphate-mediated transfection (Wigler et al., Cell 14:725-732, 1978; Corsaro and Pearson, Somatic Cell Genetics 7:603-616, 1981; Graham and Van der Eb, Virology 52d:456-467, 1973) or electroporation (Neumann et al., EMBO J. 1:841-845, 1982). To identify and select cells that express the exogenous DNA, a gene that confers a selectable phenotype (a selectable marker) is generally introduced into cells along with the gene or cDNA of interest. Preferred selectable markers include genes that confer resistance to drugs such as neomycin, hygromycin, and methotrexate. The selectable marker may be an amplifiable selectable marker. A preferred amplifiable selectable marker is a dihydrofolate reductase (DHFR) sequence. Selectable markers are reviewed by Thilly (Mammalian Cell Technology, Butterworth Publishers, Stoneham, MA, incorporated herein by reference). The person skilled in the art will easily be able to choose suitable selectable markers.

Selectable markers may be introduced into the cell on a separate plasmid at the same time as the gene of interest, or they may be introduced on the same plasmid. If, on the same plasmid, the selectable marker and the gene of interest may be under the control of different promoters or the same promoter, the latter arrangement producing a dicistronic message. Constructs of this type are known in the art (for example, Levinson and Simonsen, U.S. 4,713,339). It may also be advantageous to add additional DNA, known as "carrier DNA," to the mixture that is introduced into the cells.

After the cells have taken up the DNA, they are grown in an appropriate growth medium, typically 1-2 days, to begin expressing the gene of interest. As used herein the term "appropriate growth medium" means a medium containing nutrients and other components required for the growth of cells and the expression of the human Factor VII polypeptide variants of interest. Media generally include a carbon source, a nitrogen source, essential amino acids, essential sugars, vitamins, salts, phospholipids, protein and growth factors. For production of gamma-carboxylated proteins, the medium will contain vitamin K, preferably at a concentration of about 0.1 $\mu$g/ml to about 5 $\mu$g/ml. Drug selection is then applied to select for the growth of cells that are expressing the selectable marker in a stable fashion. For cells that have been transfected with an amplifiable selectable marker the drug concentration may be increased to select for an increased copy number of the cloned sequences, thereby increasing expression levels. Clones of stably transfected cells are then screened for expression of the human Factor VII polypeptide variant of interest.

The host cell into which the DNA sequences encoding the human Factor VII polypeptide variants is introduced may be any cell, which is capable of producing the posttranslational modified human Factor VII polypeptide variants and includes yeast, fungi and higher eucaryotic cells.

Examples of mammalian cell lines for use in the present invention are the COS-1 (ATCC CRL 1650), baby hamster kidney (BHK) and 293 (ATCC CRL 1573; Graham et al., J. Gen. Virol. 36:59-72, 1977) cell lines. A preferred BHK cell line is the tk- ts13 BHK cell line (Waechter and Baserga, Proc. Natl. Acad. Sci. USA 79:1106-1110, 1982, incorporated herein by reference), hereinafter referred to as BHK 570 cells. The BHK 570 cell line has been deposited with the American Type Culture Collection, 12301 Parklawn Dr., Rockville, Md. 20852, under ATCC accession number CRL 10314. A tk- ts13 BHK cell line is also available from the ATCC under accession number CRL 1632. In addition, a number of other cell lines may be used within the present invention, including Rat Hep I (Rat hepatoma; ATCC CRL 1600), Rat Hep II (Rat hepatoma; ATCC CRL 1548), TCMK (ATCC CCL 139), Human lung (ATCC HB 8065), NCTC 1469 (ATCC CCL 9.1), CHO (ATCC CCL 61) and DUKX cells (Urlaub and Chasin, Proc. Natl. Acad. Sci. USA 77: 4216-4220, 1980).

Examples of suitable yeasts cells include cells of *Saccharomyces* spp. or *Schizosaccharomyces* spp., in particular strains of *Saccharomyces cerevisiae* or *Saccharomyces kluyveri.* Methods for transforming yeast cells with heterologous DNA and producing heterologous polypeptides there from are described, e.g. in US 4,599,311, US 4,931,373, US 4,870,008, 5,037,743, and US 4,845,075, all of which are hereby incorporated by reference. Transformed cells are selected by a phenotype determined by a selectable marker, commonly drug resistance or the ability to grow in the absence of a particular nutrient, e.g. leucine. A preferred vector for use in yeast is the POT1 vector disclosed in US 4,931,373. The DNA sequences encoding the human Factor VII polypeptide variants may be preceded by a signal sequence and optionally a leader sequence, e.g. as described above. Further examples of suitable yeast cells are strains of *Kluyveromyces,* such as *K. lactis, Hansenula,* e.g. H. *polymorpha,* or *Pichia,* e.g. *P. pastoris* (cf. Gleeson et al., J. Gen. Microbiol. 132, 1986, pp. 3459-3465; US 4,882,279).

Examples of other fungal cells are cells of filamentous fungi, e.g. *Aspergillus* spp., *Neurospora* spp., *Fusarium* spp. or *Trichoderma* spp., in particular strains of A. *oryzae, A. nidulans* or *A. niger.* The use of *Aspergillus* spp. for the expression of proteins is described in, e.g., EP 272 277, EP 238 023, EP 184 438 The transformation of *F. oxysporum* may, for instance, be carried out as described by Malardier et al., 1989, Gene 78: 147-156. The transformation of *Trichoderma* spp. may be performed for instance as described in EP 244 234.

When a filamentous fungus is used as the host cell, it may be transformed with the DNA construct of the invention, conveniently by integrating the DNA construct in the host chromosome to obtain a recombinant host cell. This integration is generally considered to be an advantage as the DNA sequence is more likely to be stably maintained in the cell. Integration of the DNA constructs into the host chromosome may be performed according to conventional methods, e.g. by homologous or heterologous recombination.

Transformation of insect cells and production of heterologous polypeptides therein may be performed as described in US 4,745,051; US 4,879,236; US 5,155,037; 5,162,222; EP 397,485) all of which are incorporated herein by reference. The insect cell line used as the host may suitably be a *Lepidoptera* cell line, such as *Spodoptera frugiperda* cells or *Trichoplusia ni* cells (cf. US 5,077,214). Culture conditions may suitably be as described in, for instance, WO 89/01029 or WO 89/01028, or any of the aforementioned references.

The transformed or transfected host cell described above is then cultured in a suitable nutrient medium under conditions permitting expression of the human Factor VII polypeptide variant after which all or part of the resulting peptide may be recovered from the culture. The medium used to culture the cells may be any conventional medium suitable for growing the host cells, such as minimal or complex media containing appropriate supplements. Suitable media are available from commercial suppliers or may be prepared according to published recipes (e.g. in catalogues of the American Type Culture Collection). The human Factor VII polypeptide variant produced by the cells may then be recovered from the culture medium by conventional procedures including separating the host cells from the medium by centrifugation or filtration, precipitating the proteinaqueous components of the supernatant or filtrate by means of a salt, e.g. ammonium sulphate, purification by a variety of chromatographic procedures, e.g. ion exchange chromatography, gelfiltration chromatography, affinity chromatography, or the like, dependent on the type of polypeptide in question.

Transgenic animal technology may be employed to produce the Factor VII polypeptide variants of the invention. It is preferred to produce the proteins within the mammary glands of a host female mammal. Expression in the mammary gland and subsequent secretion of the protein of interest into the milk overcomes many difficulties encountered in isolating proteins from other sources. Milk is readily collected, available in large quantities, and biochemically well characterized. Furthermore, the major milk proteins are present in milk at high concentrations (typically from about 1 to 15 g/l).

From a commercial point of view, it is clearly preferable to use as the host a species that has a large milk yield. While smaller animals such as mice and rats can be used (and are preferred at the proof of principle stage), it is preferred to use livestock mammals including, but not limited to, pigs, goats, sheep and cattle. Sheep are particularly preferred due to such factors as the previous history of transgenesis in this species, milk yield, cost and the ready availability of equipment for collecting sheep milk (see, for example, WO 88/00239 for a comparison of factors influencing the choice of host species). It is generally desirable to select a breed of host animal that has been bred for dairy use, such as East Friesland sheep, or to introduce dairy stock by breeding of the transgenic line at a later date. In any event, animals of known, good health status should be used.

To obtain expression in the mammary gland, a transcription promoter from a milk protein gene is used. Milk protein genes include those genes encoding caseins (see U.S. 5,304,489), beta-lactoglobulin, $\alpha$-lactalbumin, and whey acidic protein. The beta-lactoglobulin (BLG) promoter is preferred. In the case of the ovine beta-lactoglobulin gene, a region of at least the proximal 406 bp of 5' flanking sequence of the gene will generally be used, although larger portions of the 5' flanking sequence, up to about 5 kbp, are preferred, such as a ~4.25 kbp DNA segment encompassing the 5' flanking promoter and non-coding portion of the beta-lactoglobulin gene (see Whitelaw et al., Biochem. J. 286: 31-39 (1992)). Similar fragments of promoter DNA from other species are also suitable.

Other regions of the beta-lactoglobulin gene may also be incorporated in constructs, as may genomic regions of the gene to be expressed. It is generally accepted in the art that constructs lacking introns, for example, express poorly in comparison with those that contain such DNA sequences (see Brinster et al., Proc. Natl. Acad. Sci. USA 85: 836-840 (1988); Palmiter et al., Proc. Natl. Acad. Sci. USA 88: 478-482 (1991); Whitelaw et al., Transgenic Res. 1: 3-13 (1991); WO 89/01343; and WO 91/02318, each of which is incorporated herein by reference). In this regard, it is generally preferred, where possible, to use genomic sequences containing all or some of the native introns of a gene encoding the protein or polypeptide of interest, thus the further inclusion of at least some introns from, e.g, the beta-lactoglobulin gene, is preferred. One such region is a DNA segment that provides for intron splicing and RNA polyadenylation from the 3' non-coding region of the ovine beta-lactoglobulin gene. When substituted for the natural 3' non-coding sequences of a gene, this ovine beta-lactoglobulin segment can both enhance and stabilize expression levels of the protein or polypeptide of interest. Within other embodiments, the region surrounding the initiation ATG of the variant Factor VII sequence is replaced with corresponding sequences from a milk specific protein gene. Such replacement provides a putative tissue-specific initiation environment to enhance expression. It is convenient to replace the entire variant Factor VII pre-pro and 5' non-coding sequences with those of, for example, the BLG gene, although smaller regions may be replaced.

For expression of Factor VII polypeptide variants in transgenic animals, a DNA segment encoding variant Factor VII is operably linked to additional DNA segments required for its expression to produce expression units. Such additional segments include the above-mentioned promoter, as well as sequences that provide for termination of transcription and polyadenylation of mRNA. The expression units will further include a DNA segment encoding a secretory signal sequence operably linked to the segment encoding modified Factor VII. The secretory signal sequence may be a native Factor VII secretory signal sequence or may be that of another protein, such as a milk protein (see, for example, von Heijne, Nucl. Acids Res. 14: 4683-4690 (1986); and Meade et al., U.S. 4,873,316, which are incorporated herein by reference).

Construction of expression units for use in transgenic animals is conveniently carried out by inserting a variant Factor VII sequence into a plasmid or phage vector containing the additional DNA segments, although the expression unit may be constructed by essentially any sequence of ligations. It is particularly convenient to provide a vector containing a DNA segment encoding a milk protein and to replace the coding sequence for the milk protein with that of a Factor VII variant; thereby creating a gene fusion that includes the expression control sequences of the milk protein gene. In any event, cloning of the expression units in plasmids or other vectors facilitates the amplification of the variant Factor VII sequence. Amplification is conveniently carried out in bacterial (e.g. E. coli) host cells, thus the vectors will typically include an origin of replication and a selectable marker functional in bacterial host cells. The expression unit is then introduced into fertilized eggs (including early-stage embryos) of the chosen host species. Introduction of heterologous DNA can be accomplished by one of several routes, including microinjection (e.g. U.S. Patent No. 4,873,191), retroviral infection (Jaenisch, Science 240: 1468-1474 (1988)) or site-directed integration using embryonic stem (ES) cells (reviewed by Bradley et al., Bio/Technology 10: 534-539 (1992)). The eggs are then implanted into the oviducts or uteri of pseudopregnant females and allowed to develop to term. Offspring carrying the introduced DNA in their germ line can pass the DNA on to their progeny in the normal, Mendelian fashion, allowing the development of transgenic herds. General procedures for producing transgenic animals are known in the art (see, for example, Hogan et al., Manipulating the Mouse Embryo: A Laboratory Manual, Cold Spring Harbor Laboratory, 1986; Simons et al., Bio/Technology 6: 179-183 (1988); Wall et al., Biol. Reprod. 32: 645-651 (1985); Buhler et al., Bio/Technology 8: 140-143 (1990); Ebert et al., Bio/Technology 9: 835-838 (1991); Krimpenfort et al., Bio/Technology 9: 844-847 (1991); Wall et al., J. Cell. Biochem. 49: 113-120 (1992); U.S. 4,873,191; U.S. 4,873,316; WO 88/00239, WO 90/05188, WO 92/11757; and GB 87/00458). Techniques for introducing foreign DNA sequences into mammals and their germ cells were originally developed in the mouse (see, e.g., Gordon et al., Proc. Natl. Acad. Sci. USA 77: 7380-7384 (1980); Gordon and Ruddle, Science 214: 1244-1246 (1981); Palmiter and Brinster, Cell 41: 343-345 (1985); Brinster et al., Proc. Natl. Acad. Sci. USA 82: 4438-4442 (1985); and Hogan et al. (ibid.)). These techniques were subsequently adapted for use with larger animals, including livestock species (see, e.g., WO 88/00239, WO 90/05188, and WO 92/11757; and Simons et al., Bio/Technology 6: 179-183 (1988)). To summarise, in the most efficient route used to date in the generation of transgenic mice or livestock, several hundred linear molecules of the DNA of interest are injected into one of the pro-nuclei of a fertilized egg according to established techniques. Injection of DNA into the cytoplasm of a zygote can also be employed.

Production in transgenic plants may also be employed. Expression may be generalised or directed to a particular organ, such as a tuber (see, Hiatt, Nature 344:469-479 (1990); Edelbaum et al., J. Interferon Res. 12:449-453 (1992); Sijmons et al., Bio/Technology 8:217-221 (1990); and EP 0 255 378).

The Factor VII polypeptide variants of the invention are recovered from cell culture medium or milk. The Factor VII polypeptide variants of the present invention may be purified by a variety of procedures known in the art including, but not limited to, chromatography (*e.g.*, ion exchange, affinity, hydrophobic, chromatofocusing, and size exclusion), electrophoretic procedures (*e.g.*, preparative isoelectric focusing (IEF), differential solubility (*e.g.*, ammonium sulfate precipitation), or extraction (see, *e.g.,* Protein Purification, J.-C. Janson and Lars Ryden, editors, VCH Publishers, New York, 1989). Preferably, they may be purified by affinity chromatography on an anti-Factor VII antibody column. The use of calcium-dependent monoclonal antibodies, as described by Wakabayashi et al., J. Biol. Chem. 261:11097-11108, (1986) and Thim et al., Biochemistry 27: 7785-7793, (1988), is particularly preferred. Additional purification may be achieved by conventional chemical purification means, such as high performance liquid chromatography. Other methods of purification, including barium citrate precipitation, are known in the art, and may be applied to the purification of the novel Factor VII polypeptide variants described herein (see, for example, Scopes, R., Protein Purification, Springer-Verlag, N.Y., 1982).

For therapeutic purposes it is preferred that the Factor VII polypeptide variants of the invention are substantially pure. Thus, in a preferred embodiment of the invention the Factor VII polypeptide variants of the invention is purified to at least about 90 to 95% homogeneity, preferably to at least about 98% homogeneity. Purity may be assessed by e.g. gel electrophoresis and amino-terminal amino acid sequencing.

The Factor VII variant is cleaved at its activation site in order to convert it to its two-chain form. Activation may be carried out according to procedures known in the art, such as those disclosed by Osterud, et al., Biochemistry 11: 2853-2857 (1972); Thomas, U.S. Patent No. 4,456,591; Hedner and Kisiel, J. Clin. Invest. 71:1836-1841 (1983); or Kisiel and Fujikawa, Behring Inst. Mitt. 73:29-42 (1983). Alternatively, as described by Bjoern et al. (Research Disclosure, 269 September 1986, pp. 564-565), Factor VII may be activated by passing it through an ion-exchange chromatography column, such as Mono Q® (Pharmacia fine Chemicals) or the like. The resulting activated Factor VII variant may then be formulated and administered as described below.

## ASSAYS

The invention also provides suitable assays for selecting preferred Factor VIIa variants according to the invention. These assays can be performed as a simple preliminary *in vitro* test.

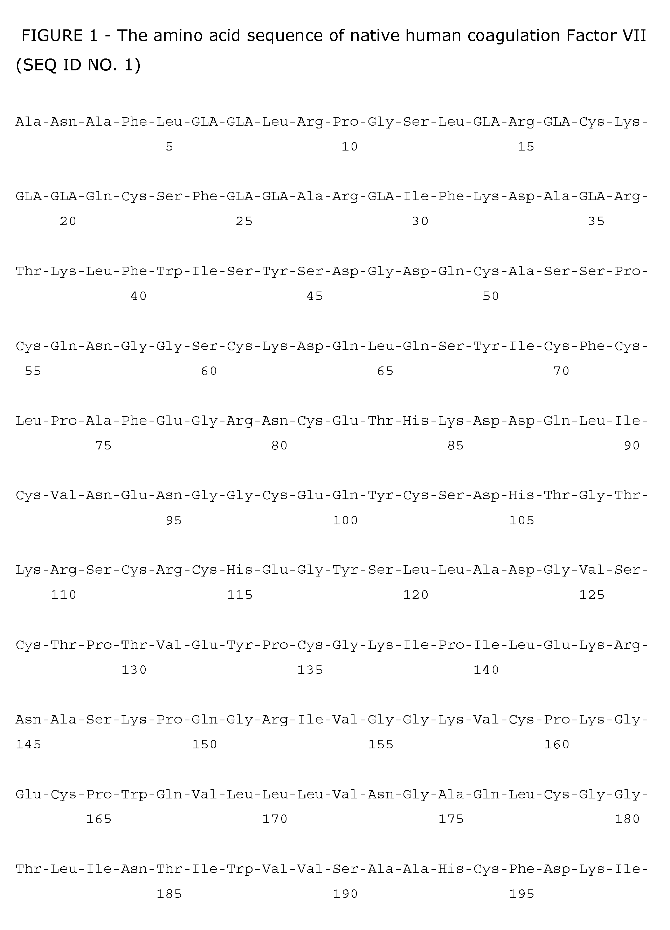

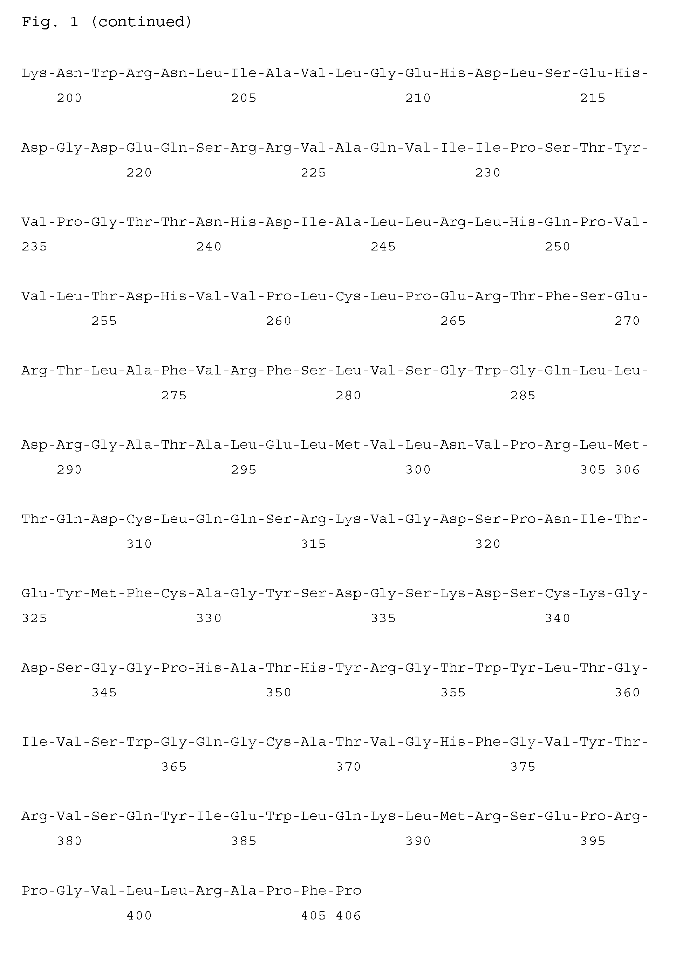

Thus, Example 4 herein discloses a simple test (entitled *"In Vitro* Hydrolysis Assay") for the activity of Factor VIIa variants of the invention. Based thereon, Factor VIIa variants which are of particular interest are such variants where the ratio between the activity of the variant and the activity of native Factor VII shown in Fig. 1 is above 1.0, e.g. at least about 1.25, preferably at least about 2.0, such as at least about 3.0 or, even more preferred, at least about 4.0 when tested in the *"In Vitro* Hydrolysis Assay".

The activity of the variants can also be measured using a physiological substrate such as factor X *("In Vitro* Proteolysis Assay")* (see Example 5), suitably at a concentration of 100-1000 nM, where the factor Xa generated is measured after the addition of a suitable chromogenic substrate (eg. S-2765). In addition, the activity assay may be run at physiological temperature.

The ability of the Factor VIIa variants to generate thrombin can also be measured in an assay comprising all relevant coagulation factors and inhibitors at physiological concentrations (minus factor VIII when mimicking hemophilia A conditions) and activated platelets (as described on p. 543 in Monroe et al. (1997) Brit. J. Haematol. 99, 542-547 which is hereby incorporated as reference).

Administration and pharmaceutical compositions

The Factor VII polypeptide variants according to the present invention may be used to control bleeding disorders which have several causes such as clotting factor deficiencies (e.g. haemophilia A and B or deficiency of coagulation factors XI or VII) or clotting factor inhibitors, or they may be used to control excessive bleeding occurring in subjects with a normally functioning blood clotting cascade (no clotting factor deficiencies or inhibitors against any of the coagulation factors). The bleedings may be caused by a defective platelet function, thrombocytopenia or von Willebrand's disease. They may also be seen in subjects in whom an increased fibrinolytic activity has been induced by various stimuli.

In subjects who experience extensive tissue damage in association with surgery or vast trauma, the haemostatic mechanism may be overwhelmed by the demand of immediate haemostasis and they may develop bleedings in spite of a normal haemostatic mechanism. Achieving satisfactory haemostasis is also a problem when bleedings occur in organs such as the brain, inner ear region and eyes and may also be a problem in cases of diffuse bleedings (haemorrhagic gastritis and profuse uterine bleeding) when it is difficult to identify the source. The same problem may arise in the process of taking biopsies from various organs (liver, lung, tumour tissue, gastrointestinal tract) as well as in laparoscopic surgery. These situations share the difficulty of providing haemostasis by surgical techniques (sutures, clips, etc.). Acute and profuse bleedings may also occur in subjects on anticoagulant therapy in whom a defective haemostasis has been induced by the therapy given. Such subjects may need surgical interventions in case the anticoagulant effect has to be counteracted rapidly. Another situation that may cause problems in the case of unsatisfactory haemostasis is when subjects with a normal haemostatic mechanism are given anticoagulant therapy to prevent thromboembolic disease. Such therapy may include heparin, other forms of proteoglycans, warfarin or other forms of vitamin K-antagonists as well as aspirin and other platelet aggregation inhibitors.

A systemic activation of the coagulation cascade may lead to disseminated intravascular coagulation (DIC). However, such complications have not been seen in subjects treated with high doses of recombinant Factor VIIa because of a localised haemostatic process of the kind induced by the complex formation between Factor VIIa and TF exposed at the site of vessel wall injury. The Factor VII polypeptide variants according to the invention may thus also be used in their activated form to control such excessive bleedings associated with a normal haemostatic mechanism.

For treatment in connection with deliberate interventions, the Factor VII polypeptide variants of the invention will typically be administered within about 24 hours prior to performing the intervention, and for as much as 7 days or more thereafter. Administration as a coagulant can be by a variety of routes as described herein.

The dose of the Factor VII polypeptide variants ranges from about 0.05 mg to 500 mg/day, preferably from about 1 mg to 200 mg/day, and more preferably from about 10 mg to about 175 mg/day for a 70 kg subject as loading and maintenance doses, depending on the weight of the subject and the severity of the condition.

The pharmaceutical compositions are primarily intended for parenteral administration for prophylactic and/or therapeutic treatment. Preferably, the pharmaceutical compositions are administered parenterally, i.e., intravenously, subcutaneously, or intramuscularly, or it may be administered by continuous or pulsatile infusion. The compositions for parenteral administration comprise the Factor VII variant of the invention in combination with, preferably dissolved in, a pharmaceutically acceptable carrier, preferably an aqueous carrier. A variety of aqueous carriers may be used, such as water, buffered water, 0.4% saline, 0.3% glycine and the like. The Factor VII polypeptide variants of the invention can also be formulated into liposome preparations for delivery or targeting to the sites of injury. Liposome preparations are generally described in, e.g., U.S. 4,837,028, U.S. 4,501,728, and U.S. 4,975,282. The compositions may be sterilised by conventional, well-known sterilisation techniques. The resulting aqueous solutions may be packaged for use or filtered under aseptic conditions and lyophilised, the lyophilised preparation being combined with a sterile aqueous solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, etc.

The concentration of Factor VII variant in these formulations can vary widely, i.e., from less than about 0.5% by weight, usually at or at least about 1% by weight to as much as 15 or 20% by weight and will be selected primarily by fluid volumes, viscosities, etc., in accordance with the particular mode of administration selected.

Thus, a typical pharmaceutical composition for intravenous infusion can be made up to contain 250 ml of sterile Ringer's solution and 10 mg of the Factor VII variant. Actual methods for preparing parenterally administrable compositions will be known or apparent to those skilled in the art and are described in more detail in, for example, Remington's Pharmaceutical Sciences, 18th ed., Mack Publishing Company, Easton, PA (1990).

The compositions containing the Factor VII polypeptide variants of the present invention can be administered for prophylactic and/or therapeutic treatments. In therapeutic applications, compositions are administered to a subject already suffering from a disease, as described above, in an amount sufficient to cure, alleviate or partially arrest the disease and its complications. An amount adequate to accomplish this is defined as "therapeutically effective amount". As will be understood by the person skilled in the art amounts effective for this purpose will depend on the severity of the disease or injury as well as the weight and general state of the subject. In general, however, the effective amount will range from about 0.05 mg up to about 500 mg of the Factor VII variant per day for a 70 kg subject, with dosages of from about 1.0 mg to about 200 mg of the Factor VII variant per day being more commonly used.

The FVIIa polypeptides of the present invention may generally be employed in serious disease or injury states, that is, life threatening or potentially life threatening situations. In such cases, in view of the minimisation of extraneous substances and general lack of immunogenicity of human Factor VII polypeptide variants in humans, it may be felt desirable by the treating physician to administer a substantial excess of these variant Factor VII compositions.

In prophylactic applications, compositions containing the Factor VII variant of the invention are administered to a subject susceptible to or otherwise at risk of a disease state or injury to enhance the subject's own coagulative capability. Such an amount is defined to be a "prophylactically effective dose." In prophylactic applications, the precise amounts once again depend on the subject's state of health and weight, but the dose generally ranges from about 0.05 mg to about 500 mg per day for a 70-kilogram subject, more commonly from about 1.0 mg to about 200 mg per day for a 70-kilogram subject.

Single or multiple administrations of the compositions can be carried out with dose levels and patterns being selected by the treating physician. For ambulatory subjects requiring daily maintenance levels, the Factor VII polypeptide variants may be administered by continuous infusion using e.g. a portable pump system.

Local delivery of the Factor VII variant of the present invention, such as, for example, topical application may be carried out, for example, by means of a spray, perfusion, double balloon catheters, stent, incorporated into vascular grafts or stents, hydrogels used to coat balloon catheters, or other well established methods. In any event, the pharmaceutical compositions should provide a quantity of Factor VII variant sufficient to effectively treat the subject.

The present invention is further illustrated by the following examples which, however, are not to be construed as limiting the scope of protection. The features disclosed in the foregoing description and in the following examples may, both separately and in any combination thereof, be material for realising the invention in diverse forms thereof.

## BRIEF DESCRIPTION OF THE DRAWINGS

Figure 1. shows the full amino acid sequence of native (wild type) human coagulation Factor VII (SEQ ID NO:1).
Figure 2. The graph illustrates the loss of activity upon ATIII inhibition (relative to WT FVIIa) plotted against the FX activation data (relative to WT FVIIa) for all the FVIIa mutants generated according to example 7. The coloured box marks the area of the plot with FVIIa mutants having high proteolytic activity and slow inhibition by ATIII. WT FVIIa is marked by a filled black circle. The filled grey/red circles indicate the four most interesting FVIIa mutants.

## EXAMPLES

Example 1

Site-directed mutagenesis:

In the following, both FVIIa numbering and chymotrypsin numbering may be used. The chymotrypsin numbering is written in parentheses after the FVIIa numbering and is marked with c and the residue number, e.g. Asp289 (c146). Residues important in the substrate specificity of FVIIa were identified by site-directed mutagenesis.

Mutations were introduced in the FVII gene using QuikChange® II Site-Directed Mutagenesis Kit (Stratagene) according to the manufacturers' recommendations. Briefly, PCR-reactions contained 25 ng of plasmid pLN174, 10 pmol of each mutagenic oligonucleotide primer, 5 $\mu$l of 10$\times$ reaction buffer, 1 $\mu$l of dNTP mix, and 1 $\mu$l of *PfuUltra* High-Fidelity DNA polymerase (2.5 U/$\mu$l) in a total volume of 50 $\mu$l. The PCR conditions consisted of 30 seconds of heating at 95°C followed by 18 cycles consisting of 30 seconds at 95°C, an annealing step for 1 minute at 55°C and an extension step for 7 minutes at 68°C. Following amplification, 1 $\mu$l of *Dpn*I (10 U/$\mu$l) was added and the PCR-tubes were incubated for 1 hour at 37°C in order to digest non-mutated supercoiled double stranded DNA. *Dpn*I-treated DNA (1 $\mu$l) was transformed into 50 $\mu$l of XL1-Blue supercompetent cells by the heat-shock procedure and then plated on LB agar plates containing 100 $\mu$g/ml carbenicillin. All mutant plasmids were sequenced to verify the mutations.

Mutagenic primers from MWG-Biotech AG (Germany):

oT239I-f: CGTACGTCCCGGGCACCATCAACCACGACATCGCG
oT239I-r: CGCGATGTCGTGGTTGATGGTGCCCGGGACGTACG
oT239Y-f: CGTACGTCCCGGGCACCTACAACCACGACATCGCG
oT239Y-r: CGCGATGTCGTGGTTGTAGGTGCCCGGGACGTACG
oD289E-f: GCTGGGGGCCAGCTGCTGGAACGTGGCGCCACGGCC
oD289E-r: GGCCGTGGCGCCACGTTCCAGCAGCTGGCCCCAGC
oD289R-f: GCTGGGGGCCAGCTGCTGAGACGTGGCGCCACGGCC
oD289R-r: GGCCGTGGCGCCACGTCTCAGCAGCTGGCCCCAGC
oK341A-f: GCAGCAAGGACTCCTGCGCAGGGGACAGTGGAGGCCC
oK341A-r: GGGCCTCCACTGTCCCCTGCGCAGGAGTCCTTGCTGC
oK341E-f: GCAGCAAGGACTCCTGCGAAGGGGACAGTGGAGGCCC
oK341E-r: GGGCCTCCACTGTCCCCTTCGCAGGAGTCCTTGCTGC
oK341Q-f: GCAGCAAGGACTCCTGCCAAGGGGACAGTGGAGGCCC
oK341Q-r: GGGCCTCCACTGTCCCCTTGGCAGGAGTCCTTGCTGC

oM327N-f: CCCCAAATATCACGGAGTACAACTTCTGTGCCGGC
oM327N-r: GCCGGCACAGAAGTTGTACTCCGTGATATTTGGGG
oM327Q-f: CCCCAAATATCACGGAGTACCAGTTCTGTGCCGGC
oM327Q-r: GCCGGCACAGAACTGGTACTCCGTGATATTTGGGG
oS363A-f: CCTGACGGGCATCGTCGCCTGGGGCCAGGGC
oS363A-r: GCCCTGGCCCCAGGCGACGATGCCCGTCAGG
oS363M.f: CCTGACGGGCATCGTCATGTGGGGCCAGGGC
oS363M-r: GCCCTGGCCCCACATGACGATGCCCGTCAGG
oW364H-f: GACGGGCATCGTCAGCCACGGCCAGGGCTGCGC
oW364H-r: GCGCAGCCCTGGCCGTGGCTGACGATGCCCGTC
oQ366E-f: GGCATCGTCAGCTGGGGCGAAGGCTGCGCAACCG
oQ366E-r: CGGTTGCGCAGCCTTCGCCCCAGCTGACGATGCC

Example 2

Mammalian expression of FVII mutants:

Baby Hamster Kidney cells (BHK) were transfected with 1.5 $\mu$g DNA of each mutant FVII expression plasmid. $5 \cdot 10^6$ BHK-cells were seeded in a T175 Nunc Easy Flask with culture medium (Dubeccos Modified Eagles medium (DMEM) with glutamax-1 from Gibco (containing sodium pyruvate, pyridoxine and 4500 g/L glucose), 10% Fetal Bovine Serum (FBS) and 1% penicillin and streptomycin). After three days of incubation in the $CO_2$-incubator, the cells were trypsinated and $0.5 \cdot 10^6$ cells per T25 Nunc Easy Flask were seeded in 5 ml of culture medium.

The FVII mutants were transfected using the FuGene™ 6 Transfection Reagent from Roche. 155 $\mu$l of DMEM was mixed with 3 $\mu$l of FuGene6 Transfection Reagent in a small cryo tube and incubated for 5 minutes at room temperature. 1.5 $\mu$g DNA of each mutant was pipetted into a new cryo tube and the DMEM-FuGene6 transfection mix was added drop wise to the DNA. After 15 minutes of incubation at room temperature the FuGene6/DMEM/DNA mixture was added drop wise to a T25 Nunc flask seeded with BHK cells. An extra T25 flask not transfected was included as control.

After over night incubation in the $CO_2$-incubator the media was changed to selection media (DMEM, 10% FBS, 1% penicillin and streptomycin and 1 $\mu$M methotrexate (MTX)). MTX is an inhibitor of dihydrofolate reductase. Only transfected cells are expected to survive the MTX treatment since the mutant FVII plasmids express highly elevated levels of dihydrofolate reductase thereby selecting for MTX-resistant cells. The selection media was changed approximately every second day for 2 weeks. At confluence, the cells were trypsinated and transferred to T175 flasks with 30 ml of selection medium. After 2-3 days of incubation in the $CO_2$-incubator, the cells were trypsinated and transferred to $1 \times 3$ layer cell factories with 100 ml of culture medium (DMEM, 10% FBS, 1% Penicillin and Streptomycin). At confluence, the media was removed and 100 ml of production media (DMEM, 2% FBS, 1% Penicillin and Streptomycin and 2.5 $\mu$l of Vitamin K per 500 ml DMEM) was added to each cell factory. After two days of incubation the supernatants were harvested and 100 ml of new production media was added to each $1 \times 3$ layer flask. Harvest and addition of new production media were done 3 times pr. week (total 5 times).

Example 3

Purification of FVII mutants:

The FVII mutants were purified by a two-step procedure using an ÄKTA Explorer from Amersham Biosciences:

1. Ion exchange chromatography using a Q-Sepharose Fast Flow column (anion exchanger) ~ 50 ml (Amersham Biosciences)
2. Affinity chromatography using a F1A2 Sepharose 4B anti-FVII Antibody Column ~ 10 ml.

The five harvested supernatants from each mutant were pooled and adjusted to pH 8 and a final concentration of 10 mM Tris and 5 mM EDTA. The conductivity was adjusted to $\lambda = 11.6$ mS/cm with deionised water. The supernatants were applied to the Q-Sepharose Fast Flow column equilibrated with 10 mM Tris, 50 mM NaCl pH 8.0. FVII has an isoelectric point of 7 at pH= 8 allowing FVII to bind to the Q-Sepharose column. After washing with equilibration buffer until baseline was low, the proteins were eluted with a linear gradient with 10 mM Tris, 50 mM NaCl, 25 mM $CaCl_2$ pH 8.0. The top fractions were pooled and adjusted to pH 7.5 and applied to the antibody column equilibrated with 50 mM Hepes, 100 mM NaCl, 10 mM $CaCl_2$ pH 7.5. The column was washed with equilibration buffer until baseline was low and high NaCl buffer (50 mM Hepes, 2 M NaCl, 10 mM $CaCl_2$, pH 7.5) was run on the column followed by wash with equilibration buffer. The proteins were eluted with a linear gradient with 50 mM Hepes, 100 mM NaCl, 10 mM EDTA pH 7.5. The top fractions were pooled and 1 M CaCl$_2$ was added to a final concentration of 15 mM. The purified mutants were concentrated to a volume of ~1 ml with a Millicon15 from Millipore removing components below 15000 Da. The FVII mutants were left for auto-activation at room temperature for a few days. The mutants that were unable to auto-activate as elucidated by SDS-PAGE, were activated by immobilised FXa by coupling FXa to CnBr activated Sepharose from Amersham Biosciences.

Example 4

Amidolytic activity of FVIIa mutants:

The amidolytic activity of mutant FVIIa towards the chromogenic substrate S-2288 (Chromogenix) was assayed at substrate concentrations ranging from 0.2-10 mM in buffer containing 50 mM HEPES, pH 7.4, 100 mM NaCl, 5 mM CaCl$_2$, 1 mg/ml BSA. Reactions were initiated by addition of enzyme to final concentrations of 100 nM and 10 nM in the absence or presence of 100 nM sTF, respectively. Product formation was monitored continuously at 405 nm in a Spectramax 340 Microplate spectrophotometer. Initial velocities were plotted against the substrate concentrations and the data were fitted to the Michaelis-Menten equation using non-linear regression analysis to obtain the $k_{cat}$ and $K_M$ values. When $K_M$ >5 mM, only $k_{cat}/K_M$ were determined by linear fit of data at low substrate concentrations. A standard curve generated with concentrations of pNA from 0.06-0.6 mM was used to convert the absorbance units into molar concentrations.

**Results:**

Amidolytic activity as measured by the "*In Vitro* Hydrolysis Assay":

S-2288 hydrolysis:

| | $K_M$ (mM) | $k_{cat}$ (s$^{-1}$) | $k_{cat}/K_M$ (mM$^{-1}$ ·s$^{-1}$) | Fold enhancenment by sTF |
|---|---|---|---|---|
| **FVIIa** | | | 0.92 ± 0.007 | |
| **FVIIa-sTF** | 1.27 ± 0.13 | 33.17 ± 0.91 | 26.12 ± 2.77 | 28.39 |
| **T238A-sTF** | | | 37.71 ± 2.82 | |
| **T239A-sTF** | | | 5.45 ± 0.15 | |
| **T239G-sTF** | | | 2.40 ± 0.036 | |
| **T239I** | | | 0.29 ± 0.014 | |
| **T239I-sTF** | | 12.72 ± 0.33 | 19.71 ± 2.26 | 67.97 |
| **T239Y** | | | 0.031 ± 0.0015 | |
| **T239Y-sTF** | | | 2.47 ± 0.18 | 79.68 |
| **D289E** | | | 1.43 ± 0.09 | |
| **D289E-sTF** | 0.66 ± 0.037 | 21.93 ± 0.32 | 33.23 ± 1.93 | 23.24 |
| **K341A** | | | 0.63 ± 0.02 | |
| **K341A-sTF** | 1.45 ± 0.15 | 29.96 ± 0.98 | 20.66 ± 2.24 | 32.79 |
| **K341E** | | | 2.99 ± 0.28 | |
| **K341E-sTF** | 1.04 ± 0.13 | 23.51 ± 0.89 | 22.60 ± 2.95 | 7.56 |
| **K341Q** | | | 1.05 ± 0.05 | |
| **K341Q-sTF** | 0.81 ± 0.06 | 26.60 ± 0.54 | 32.83 ± 2.52 | 31.27 |
| **M327N** | | | 0.048 ± 0.0008 | |
| **M327N-sTF** | | | 0.97 ± 0.01 | 20.21 |

(continued)

| | $K_M$ (mM) | $k_{cat}$ (s$^{-1}$) | $k_{cat}/K_M$ (mM$^{-1}\cdot$s$^{-1}$) | Fold enhancenment by sTF |
|---|---|---|---|---|
| M327Q | | | 0.17 ± 0.003 | |
| M327Q-sTF | | | 1.79 ± 0.06 | 10.53 |
| S363M-sTF | | | 0.36 ± 0.002 | |
| W364H | | | 0.11 ± 0.002 | |
| W364H-sTF | | | 0.92 ± 0.002 | 8.36 |
| Q366E | | | 1.11 ± 0.02 | |
| Q366E-sTF | 1.46 ± 0.15 | 28.95 ± 0.89 | 19.83 ± 2.13 | 17.86 |

**Example 5**

Proteolytic activity of FVIIa mutants as measured by *"In Vitro* Proteolysis Assay"*:

Kinetic parameters of FX activation were determined using a two-stage assay. Each variant (10 nM) was incubated with 100 nM sTF and 0.1-6.4 μM FX (Enzyme Research Laboratories Ltd) in a total volume of 100 μl for 20 min at room temperature. Reactions were quenched by addition of 50 μl stop buffer (50 mM Hepes pH 7.4, 100 mM NaCl, 20 mM EDTA) and the amount of FXa generated determined by addition of 50 μl 2 mM chromogenic substrate S-2765 (Chromogenix). S-2765 cleavage was monitored at 405 nm using a Spectramax 340 Microplate spectrophotometer. The amount of FXa generated on a molar basis was estimated from standard curves using final concentrations of 0.5-10 nM FXa (Enzyme Research Laboratories Ltd). Pseudo first-order kinetics was assumed since less than 10% of the total amount of FX was converted to FXa during the assay. $k_{cat}/K_M$ values were determined by linear fits of the initial velocities.

**Results:**

Proteolytic activity:

FX activation:

| | $k_{cat}/K_M$ (M$^{-1}\cdot$s$^{-1}$) |
|---|---|
| FVIIa-sTF | 2329 ± 65 |
| T238A-sTF | 419 ± 27 |
| T239A-sTF | 69 ± 5 |
| T239G-sTF | 23 ± 0.6 |
| T239I-sTF | 1930 ± 117 |
| T239Y-sTF | 2099 ± 1 |
| D289E-sTF | 3707 ± 24 |
| K341A-sTF | 397 ± 18 |
| K341E-sTF | 24 ± 1 |
| K341Q-sTF | 509 ± 27 |
| M327Q-sTF | 515 ± 24 |
| M327N-sTF | 465 ± 37 |
| S363M-sTF | 5 ± 0.3 |
| W364H-sTF | 596 ± 18 |

(continued)

|  | $k_{cat}/K_M$ (M$^{-1}\cdot$s$^{-1}$) |
| --- | --- |
| **Q366E-sTF** | 1391 $\pm$ 10 |
| **S363M-sTF** | 112 $\pm$ 1 |

**Example 6**

Inhibition of FVIIa-sTF by ATIII:

The rate of inactivation of FVIIa and FVIIa mutants by ATIII (American Diagnostica Inc.) was measured under pseudo-first-order conditions by a continuous assay method in the presence of low molecular weight (LMW) heparin (Calbiochem) and sTF. All reagents were equilibrated at room temperature and the assay was performed in a buffer containing 50 mM Hepes pH 7.4, 100 mM NaCl, 5 mM CaCl$_2$, 1 mg/ml BSA, 0.1% PEG 8000. To maintain pseudo-first order conditions during the reaction, >10-fold molar excess of inhibitor over protease was used. Equal volumes of FVIIa (final concentration 40 nM) and sTF (final concentration 400 nM) were combined at pre-equilibrated 30 min at room temperature prior to assay initiation. In 96-well plates 20 $\mu$l of LMW Heparin (final concentration 3 $\mu$M) was mixed with 20 $\mu$l of ATIII in varying concentrations (final concentrations 50-750 nM) in a total volume of 130 $\mu$l resulting in 10-150 fold excess of ATIII over FVIIa. A blank without ATIII was included to show a linear initial rate throughout the assay. The reactions were started by addition of 50 $\mu$l of the FVIIa-sTF mixture to each well resulting in a final concentration of 5 nM FVIIa and 50 nM sTF followed by addition of 20 $\mu$l S-2288 (final concentration 200 $\mu$M). Kinetics was monitored for 30 minutes at 405 nm using a Spectramax 340 Microplate spectrophotometer to obtain progress curves at each inhibitor concentration. The progress curves were fitted to Eq. 1 to determine the $k_{obs}$ values:

$$\text{Eq. 1} \qquad [\text{Pr}]_t = [\text{Pr}]_0 + \frac{v_0}{k_{obs}}(1 - \exp(-k_{obs}t))$$

Here, $[\text{Pr}]_0$ and $[\text{Pr}]$ represent the concentration of product at time zero and t, respectively. To obtain $K_D$ and $k_{Lim}$ values Eq. 2 was fitted to a plot of the estimated $k_{obs}$ values against the inhibitor concentrations.

$$\text{Eq. 2} \qquad k_{obs} = \frac{k_{Lim}[\text{I}]_0}{K_D(1 + \frac{[\text{S}]_0}{K_M}) + [\text{I}]_0}$$

Here, $K_D$ represents the dissociation constant of the non-covalent enzyme-inhibitor Michaelis-type complex and $k_{Lim}$ is the observed rate constant for formation of the irreversible inhibitor-protease complex. The apparent second order rate constant of inhibition, $k_{inh}$, is then given by:

$$k_{inh} = \frac{k_{Lim}}{K_D}$$

First order rate constant of complex breakdown, $k_{brkdn}$:

FVIIa (500 nM) was reacted with 2000 nM of sTF, 1250 or 2500 nM of ATIII and a four fold excess of LMW Heparin over ATIII. Reactions were incubated at room temperature for an hour at which time the reaction had run to completion. Each reaction was then diluted 400, 450 and 500 times into 200 $\mu$l reaction mixtures in a 96-well plate containing a final concentration of 4 mM S-2288 and 100 μg/ml of Polybrene (heparin chelating agent from Sigma). The absorbance increase at 405 nm was measured for 12 hours at 405 nm using a Spectramax 340 Microplate spectrophotometer. The absorbance curves were plotted against the time and first-order rate constants of complex breakdown were determined by fitting the curves to a single exponential function. The observed first order rate constants (k) were converted to half lives ($T_{1/2}$) according to the relationship $k=\ln(2)/T_{1/2}$.

**Results:**

ATIII inhibition of FVIIa mutants:

Rate constants for ATIII inhibition:

| | $k_{Lim}$ (s⁻¹) | $K_D$ (M) | $k_{inh}$ (M⁻¹ · s⁻¹) | Relative inhibition ( $k_{inh}/ k_{inh(wtFVIIa)}$ |
|---|---|---|---|---|
| **FVIIa** | $0.00488 \pm 0.000315$ | $1.812 \cdot 10^{-7} \pm 3.326 \pm 10^{-8}$ | $26917 \pm 1019$ | 1.00 |
| **T238A** | $0.0074 \pm 0.00043$ | $2.39 \cdot 10^{-7} \pm 3.58 \cdot 10^{-8}$ | $30844 \pm 4947$ | 1.15 |
| **T239A** | $0.0016 \pm 0.00010$ | $2.60 \cdot 10^{-7} \pm 4.14 \cdot 10^{-8}$ | $6160 \pm 1058$ | 0.23 |
| **T239G** | $0.0015 \pm 0.00014$ | $1.14 \cdot 10^{-7} \pm 7.12 \cdot 10^{-8}$ | $4688 \pm 1155$ | 0.17 |
| **T239Y** | $0.00533 \pm 0.000294$ | $1.743 \cdot 10^{-7} \pm 2.659 \cdot 10^{-8}$ | $30591 \pm 805$ | 1.14 |
| **T239I** | $0.00254 \pm 0.000074$ | $1.678 \cdot 10^{-7} \pm 1.271 \cdot 10^{-8}$ | $15146 \pm 100$ | 0.56 |
| **D289E** | $0.00322 \pm 0.000132$ | $6.742 \cdot 10^{-8} \pm 1.155 \cdot 10^{-8}$ | $47786 \pm 1481$ | 1.78 |
| **Q366E** | $0.00255 \pm 0.000106$ | $2.489 \cdot 10^{-7} \pm 2.631 \cdot 10^{-8}$ | $10229 \pm 132$ | 0.38 |

First order rate constants and half life for breakdown of ATIII-FVIIa complexes:

| | $k_{br kdn}$ (s⁻¹) | $T_{1/2}$ (hours) |
|---|---|---|
| **FVIIa** | $3.43 \cdot 10^{-5}$ | 5.63 |
| **T238A** | $3.39 \cdot 10^{-5}$ | 5.7 |
| **T239A** | $2.85 \cdot 10^{-5}$ | 6.8 |
| **T239G** | $1.61 \cdot 10^{-5}$ | 12.0 |
| **D289E** | $9.16 \cdot 10^{-5}$ | 2.12 |
| **T239I** | $1.02 \cdot 10^{-4}$ | 1.93 |
| **T239Y** | $2.08 \cdot 10^{-5}$ | 9.32 |
| **Q366E** | $2.31 \cdot 10^{-5}$ | 8.36 |

Rate constants were measured in the presence of sTF and LMW heparin.

**Example 7**

SCREENING OF TRANSIENTLY EXPRESSED FVII MUTANTS

A high throughput system for screening of FVIIa mutants was employed based on the FreeStyle™ 293-F transient expression system from Invitrogen. An enteropeptidase cleavage site had been engineered into a FVII expression plasmid by replacing amino acids 148-151 with four Asp residues setup previously in the laboratory. To prevent further proteolysis at the Arg290-Gly291 (c147-c149) bond by enteropeptidase, the mutation D289A (c146) was also introduced into the expression plasmid. A model of the ATIII-FVIIa-sTF complex was generated from the crystal structure of a trypsin-serpin complex. The productive loop conformation of the RCL of the serpin was modelled to ATIII and the final complex was generated based on the trypsin-serpin structure. Based on the model of the ATIII-FVIIa-sTF complex, a total of 26 FVII mutants were generated in the FVII D289A (c146) enteropeptidase background by QuickChange site-directed mutagenesis (Table 3).

The FVII variants were transiently expressed in human embryonic kidney cells in serum-free expression media yielding expression levels of 2-3 mg FVII per litre media for the majority of the FVIIa variants as deduced from ELISA determinations. Expression level of V172A (c35), Q176L (c40), D196A (c60), T293L (c151), D319A (c170G) <0.2 mg per litre. The activated fraction representing functional FVIIa for each FVIIa mutant was characterized with respect to amidolytic and proteolytic activity and ATIII inhibition (Table 3).

The amidolytic activity towards S-2288 was measured in the presence of sTF. The proteolytic activity as measured by activation of FX could not be determined in the presence of sTF due to tiny amounts of cell components present in the media accelerating the auto-activation of FX by providing a phospholipid surface. Instead, FX activation was performed in the presence of relipidated TF (Innovin). The low-expression mutants (V172A (c35), Q176L (c40), D196A (c60), T293L (c151), D319A (c170G)) could only be characterized with respect to the proteolytic activity. V235A (c95) showed no activity towards S-2288; therefore only FX activation was performed for this mutant.

The majority of the FVIIa mutants had amidolytic and proteolytic activities >75% relative to WT FVIIa. Only V235A (c95), N240D (c100), T293A (c151), G367E (c219) and H373K (c224) and the low-expression mutants (V172A (c35), Q176L (c40), D196A (c60), T293L (c151), D319A (c170G)) showed proteolytic activities <60% relative to WT FVIIa. The rate of ATIII inhibition was determined by measuring the loss of amidolytic activity after 30 minutes of incubation with a 10-fold excess of ATIII relative to FVIIa in the presence of sTF and LMW heparin. The absolute loss of activity ranged from 2% for H373K (c224) to 55% for P321A (c170I).

Table 3. Kinetic analysis of WT FVIIa (D289A (c146) background) and a total of 26 FVIIa mutants expressed in a transient expression system with respect to 1) hydrolysis of 1 mM S-2288, 2) activation of 150 nM FX and 3) inhibition by 100 nM ATIII-LMW heparin. The amidolytic assay and the ATIII assay was performed in the presence of a ten fold excess of sTF relative to FVIIa. The proteolytic assay was performed in the presence of relipidated TF. All assays were performed in 50 mM Hepes, 100 mM NaCl, 5 mM CaCl$_2$, 1 mg/ml BSA, pH 7.4. Abbreviations: N.D.: Not determined because of low expression level.

| FVII numbering | Chymotrypsin numbering | Amidolytic activity Relative to WT (%) | FX activation Relative to WT (%) | ATIII inhibition | |
|---|---|---|---|---|---|
| | | | | Loss of activity (%) | Relative to WT (%) |
| **WT FVIIa** | **WT FVIIa** | 100 | 100 | 36 | 100 |
| **V172A** | **V35A** | N.D. | 19 | N.D. | N.D. |
| **N173A** | **N37A** | 121 | 90 | 22 | 61 |
| **A175K** | **A39K** | 131 | 105 | 37 | 104 |
| **Q176A** | **Q40A** | 114 | 75 | 6 | 17 |
| **Q176L** | **Q40L** | N.D. | 8 | N.D. | N.D. |
| **L177F** | **L41F** | 119 | 77 | 35 | 97 |
| **D196A** | **D60A** | N.D. | 8 | N.D. | N.D. |
| **K197A** | **K60(A)A** | 160 | 70 | 30 | 84 |
| **I198A** | **I60(B)A** | 95 | 86 | 19 | 53 |
| **K199A** | **K60(C)A** | 110 | 98 | 15 | 43 |
| **N200A** | **N60(D)A** | 92 | 91 | 35 | 99 |
| **N203A** | **V63A** | 163 | 98 | 28 | 78 |
| **V235A** | **V95A** | N.D. | 2 | N.D. | N.D. |
| **T238K** | **T98K** | 108 | 92 | 41 | 115 |
| **N240D** | **N100D** | 49 | 58 | 15 | 42 |
| **Q286A** | **Q143A** | 95 | 83 | 27 | 77 |
| **R290K** | **R147K** | 117 | 76 | 18 | 51 |

(continued)

| FVII numbering | Chymotrypsin numbering | Amidolytic activity Relative to WT (%) | FX activation Relative to WT (%) | ATIII inhibition | |
|---|---|---|---|---|---|
| | | | | Loss of activity (%) | Relative to WT (%) |
| T293A | T151A | 86 | 59 | 4 | 12 |
| T293L | T151L | N.D. | 6 | N.D. | N.D. |
| D319A | D170(G)A | N.D. | 8 | N.D. | N.D. |
| S320A | S170(H)A | 146 | 90 | 35 | 99 |
| P321A | P170(I)A | 80 | 82 | 55 | 155 |
| P321G | P170(I)G | 74 | 73 | 23 | 64 |
| G367E | G219E | 16 | 10 | 5 | 13 |
| T370A | T221A | 152 | 81 | 42 | 117 |
| H373K | H224K | 16 | 40 | 2 | 4 |

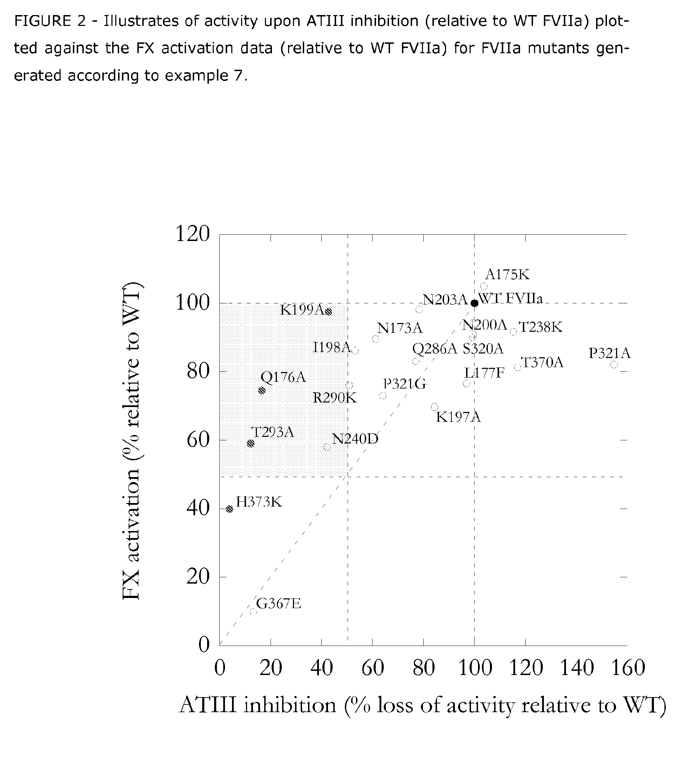

To identify FVIIa mutants able to activate FX at a reasonable rate but less prone to inhibition by ATIII, the loss of activity upon ATIII inhibition (relative to WT FVIIa) was plotted against the FX activation data (relative to WT FVIIa) for all the FVIIa mutants as illustrated in Figure 2. The coloured box in Figure 2 marks FVIIa mutants showing high proteolytic activity and slow inhibition by ATIII relative to WT FVIIa.

Embodiments according the invention:

1. A Factor VII polypeptide comprising one or more substitutions relative to the amino acid sequence of SEQ ID NO:1, wherein said substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein said Factor VII polypeptide exhibits increased resistance to inactivation by an endogenous inhibitor of said FVII polypeptide relative to wild-type human FVIIa.

2. The Factor VII polypeptide according to embodiment 1, wherein said Factor VII poly-peptide has increased functional in vivo half-life relative to human wild-type Factor VIIa.

3. The Factor VII polypeptide according to embodiments 1 or 2, wherein A175 is replaced with any other amino acid.

4. The Factor VII polypeptide according to any one of embodiments 1-3, wherein Q176 is replaced with any other amino acid.

5. The Factor VII polypeptide according to any of embodiments 1-4, wherein L177 is re-placed with any other amino acid.

6. The Factor VII polypeptide according to any of embodiments 1-5, wherein D196 is re-placed with any other amino acid.

7. The Factor VII polypeptide according to any of embodiments 1-6, wherein K197 is re-placed with any other amino acid.

8. The Factor VII polypeptide according to any of embodiments 1-7, wherein I198 is re-placed with any other amino acid.

9. The Factor VII polypeptide according to any of embodiments 1-8, wherein K199 is re-placed with any other amino acid.

10. The Factor VII polypeptide according to any of embodiments 1-9, wherein G237 is re-placed with any other amino acid.

11. The Factor VII polypeptide according to any of embodiments 1-10, wherein T238 is replaced with any other amino acid.

12. The Factor VII polypeptide according to any of embodiments 1-11, wherein T239 is replaced with any other amino acid.

13. The Factor VII polypeptide according to any of embodiments 1-12, wherein Q286 is replaced with any other amino acid.

14. The Factor VII polypeptide according to any of embodiments 1-13, wherein L287 is replaced with any other amino acid.

15. The Factor VII polypeptide according to any of embodiments 1-14, wherein L288 is replaced with any other amino acid.

16. The Factor VII polypeptide according to any of embodiments 1-15, wherein D289 is replaced with any other amino acid.

17. The Factor VII polypeptide according to any of embodiments 1-16, wherein R290 is replaced with any other amino acid.

18. The Factor VII polypeptide according to any of embodiments 1-17, wherein G291 is replaced with any other amino acid.

19. The Factor VII polypeptide according to any of embodiments 1-18, wherein A292 is replaced with any other amino acid.

20. The Factor VII polypeptide according to any of embodiments 1-19, wherein T293 is replaced with any other amino acid.

21. The Factor VII polypeptide according to any of embodiments 1-20, wherein A294 is replaced with any other amino acid.

22. The Factor VII polypeptide according to any of embodiments 1-21, wherein L295 is replaced with any other amino acid.

23. The Factor VII polypeptide according to any of embodiments 1-22, wherein L297 is replaced with any other amino acid.

24. The Factor VII polypeptide according to any of embodiments 1-23, wherein V299 is replaced with any other amino acid.

25. The Factor VII polypeptide according to any of embodiments 1-24, wherein M327 is replaced with any other amino acid.

26. The Factor VII polypeptide according to any of embodiments 1-25, wherein K341 is replaced with any other amino acid.

27. The Factor VII polypeptide according to any of embodiments 1-26, wherein S363 is replaced with any other amino acid.

28. The Factor VII polypeptide according to any of embodiments 1-27, wherein W364 is replaced with any other amino acid.

29. The Factor VII polypeptide according to any of embodiments 1-28, wherein G365 is replaced with any other amino acid.

30. The Factor VII polypeptide according to any of embodiments 1-29, wherein Q366 is replaced with any other amino acid.

31. The Factor VII polypeptide according to any of embodiments 1-30, wherein V172 is replaced with any other amino acid.

32. The Factor VII polypeptide according to any of embodiments 1-31, wherein N173 is replaced with any other amino acid.

33. The Factor VII polypeptide according to any of embodiments 1-32, wherein N200 is replaced with any other amino acid.

34. The Factor VII polypeptide according to any of embodiments 1-33, wherein N203 is replaced with any other amino acid.

35. The Factor VII polypeptide according to any of embodiments 1-34, wherein V235 is replaced with any other amino acid.

36. The Factor VII polypeptide according to any of embodiments 1-35, wherein N240 is replaced with any other amino acid.

37. The Factor VII polypeptide according to any of embodiments 1-36, wherein D319 is replaced with any other amino acid.

38. The Factor VII polypeptide according to any of embodiments 1-37, wherein S320 is replaced with any other amino acid.

39. The Factor VII polypeptide according to any of embodiments 1-38, wherein P321 is replaced with any other amino acid.

40. The Factor VII polypeptide according to any of embodiments 1-39, wherein G367 is replaced with any other amino acid.

41. The Factor VII polypeptide according to any of embodiments 1-40, wherein T370 is replaced with any other amino acid.

42. The Factor VII polypeptide according to any of embodiments 1-41, wherein H373 is replaced with any other amino acid.

43. The Factor VII polypeptide according to embodiment 12, wherein T239 is replaced with any other amino acid selected from Gly, Ala, Val, Leu, Ile, Phe, Met, Trp, Tyr, Asp, Asn, Glu, Gln, His, Lys, Arg, Cys, Ser

44. The Factor VII polypeptide according to embodiment 16, wherein D289 is replaced with any other amino acid selected from Gly, Ala, Val, Leu, Ile, Phe, Met, Trp, Tyr, Asn, Glu, Gln, His, Lys, Arg, Cys, Ser, Thr

45. The Factor VII polypeptide according to embodiment 25, wherein M327 is replaced with any other amino acid selected from Gly, Ala, Val, Leu, Ile, Phe, Trp, Tyr, Asp, Asn, Glu, Gln, His, Lys, Arg, Cys, Ser, Thr.

46. The Factor VII polypeptide according to embodiment 26, wherein K341 is replaced with any other amino acid selected from Gly, Ala, Val, Leu, Ile, Phe, Met, Trp, Tyr, Asp, Asn, Glu, Gln, His, Arg, Cys, Ser, Thr .

47. The Factor VII polypeptide according to embodiment 27, wherein S363 is replaced with any other amino acid selected from Gly, Ala, Val, Leu, Ile, Phe, Met, Trp, Tyr, Asp, Asn, Glu, Gln, His, Lys, Arg, Cys, Thr .

48. The Factor VII polypeptide according to embodiment 31, wherein V172 is replaced with any other amino acid selected from Gly, Ala, Leu, Ile, Phe, Met, Trp, Tyr, Asp, Asn, Glu, Gln, His, Lys, Arg, Cys, Ser, Thr

49. The Factor VII polypeptide according to embodiment 32, wherein N173 is replaced with any other amino acid selected from Gly, Ala, Val, Leu, Ile, Phe, Met, Trp, Tyr, Asp, Glu, Gln, His, Lys, Arg, Cys, Ser, Thr

50. The Factor VII polypeptide according to embodiment 33, wherein N200 is replaced with any other amino acid selected from Gly, Ala, Val, Leu, Ile, Phe, Met, Trp, Tyr, Asp, Glu, Gln, His, Lys, Arg, Cys, Ser, Thr

51. The Factor VII polypeptide according to embodiment 34, wherein N203 is replaced with any other amino acid selected from Gly, Ala, Val, Leu, Ile, Phe, Met, Trp, Tyr, Asp, Glu, Gln, His, Lys, Arg, Cys, Ser, Thr

52. The Factor VII polypeptide according to embodiment 35, wherein V235 is replaced with any other amino acid selected from Gly, Ala, Leu, Ile, Phe, Met, Trp, Tyr, Asp, Asn, Glu, Gln, His, Lys, Arg, Cys, Ser, Thr

53. The Factor VII polypeptide according to embodiment 36, wherein N240 is replaced with any other amino acid selected from Gly, Ala, Val, Leu, Ile, Phe, Met, Trp, Tyr, Asp, Glu, Gln, His, Lys, Arg, Cys, Ser, Thr

54. The Factor VII polypeptide according to embodiment 37, wherein D319 is replaced with any other amino acid selected from Gly, Ala, Val, Leu, Ile, Phe, Met, Trp, Tyr, Asn, Glu, Gln, His, Lys, Arg, Cys, Ser, Thr

55. The Factor VII polypeptide according to embodiment 38, wherein S320 is replaced with any other amino acid selected from Gly, Ala, Val, Leu, Ile, Phe, Met, Trp, Tyr, Asp, Asn, Glu, Gln, His, Lys, Arg, Cys, Thr

56. The Factor VII polypeptide according to embodiment 39, wherein P321 is replaced with any other amino acid selected from Gly, Ala, Val, Leu, Ile, Phe, Met, Trp, Tyr, Asp, Asn, Glu, Gln, His, Lys, Arg, Cys, Ser, Thr

57. The Factor VII polypeptide according to embodiment 40, wherein G367 is replaced with any other amino acid selected from Ala, Val, Leu, Ile, Phe, Met, Trp, Tyr, Asp, Asn, Glu, Gln, His, Lys, Arg, Cys, Ser, Thr

58. The Factor VII polypeptide according to embodiment 41, wherein T370 is replaced with any other amino acid selected from Gly, Ala, Val, Leu, Ile, Phe, Met, Trp, Tyr, Asp, Asn, Glu, Gln, His, Lys, Arg, Cys, Ser.

59. The Factor VII polypeptide according to embodiment 42, wherein H373 is replaced with any other amino acid selected from Gly, Ala, Val, Leu, Ile, Phe, Met, Trp, Tyr, Asp, Asn, Glu, Gln, Lys, Arg, Cys, Ser, Thr

60. The Factor VII polypeptide according to any of embodiments 1-59, wherein at least one amino acid in the remaining positions in the protease domain has been re-placed with any other amino acid.

61. The Factor VII polypeptide according to embodiments 60, wherein at the most 20 ad-ditional amino acids in the remaining positions in the protease domain have been replaced with any other amino acids.

62. The Factor VII polypeptide according to any of embodiments 1-61, wherein at least one amino acid corresponding to an amino acid at a position selected from 157-170 of SEQ ID NO:1 has been replaced with any other amino acid.

63. The Factor VII polypeptide according to any of embodiments 1-62, wherein at least one amino acid corresponding to an amino acid at a position selected from 290-305 of SEQ ID NO:1 has been replaced with any other amino acid.

64. The Factor VII polypeptide according to any of embodiments 1-63, wherein R304 has been replaced by an amino acid selected from the group consisting of Tyr, Phe, Leu, and Met.

65. The Factor VII polypeptide according to any of embodiments 1-64, wherein at least one amino acid corresponding to an amino acid at a position selected from 306-312 of SEQ ID NO:1 has been replaced with any other amino acid.

66. The Factor VII polypeptide according to any of embodiments 1-64, wherein M306 has been replaced by an amino acid selected from the group consisting of Asp, and Asn.

67. The Factor VII polypeptide according to any of embodiments 1-66, wherein D309 has been replaced by an amino acid selected from the group consisting of Ser, and Thr.

68. The Factor VII polypeptide according to any of embodiments 1-67, wherein at least one amino acid corresponding to an amino acid at a position selected from 330-339 of SEQ ID NO:1 has been replaced with any other amino acid.

69. The Factor VII polypeptide according to any of embodiments 1-68, wherein A274 has been replaced with any other amino acid.

70. The Factor VII polypeptide according to embodiment 69, wherein said A274 has been replaced by an amino acid selected from the group consisting of Met, Leu, Lys, and Arg.

71. The Factor VII polypeptide according to any of embodiments 1-70, wherein K157 has been replaced by an amino acid selected from the group consisting of Gly, Val, Ser, Thr, Asn, Gln, Asp, and Glu.

72. The Factor VII polypeptide according to any of embodiments 1-71, wherein K337 has been replaced by an amino acid selected from the group consisting of Ala, Gly, Val, Ser, Thr, Asn, Gln, Asp, and Glu.

73. The Factor VII polypeptide according to any of embodiments 1-72, wherein D334 has been replaced by an amino acid selected from the group consisting of Gly, and Glu.

74. The Factor VII polypeptide according to any of embodiments 1-73, wherein S336 has been replaced by an amino acid selected from the group consisting of Gly, and Glu.

75. The Factor VII polypeptide according to any of embodiments 1-74, wherein V158 has been replaced by an amino acid selected from the group consisting of Ser, Thr, Asn, Gln, Asp, and Glu.

76. The Factor VII polypeptide according to any of embodiments 1-75, wherein E296 has been replaced by an amino acid selected from the group consisting of Arg, Lys, Ile, Leu and Val.

77. The Factor VII polypeptide according to any of embodiments 1-76, wherein M298 has been replaced by an amino acid selected from the group consisting of Lys, Arg, Gln, and Asn.

78. The Factor VII polypeptide according to any of embodiments 1-77, wherein L305 has been replaced by an amino acid selected from the group consisting of Val, Tyr and Ile.

79. The Factor VII polypeptide according to any of embodiments 1-78 , wherein S314 has been replaced by an amino acid selected from the group consisting of Gly, Lys, Gln and Glu.

80. The Factor VII polypeptide according to any of embodiments 1-79, wherein F374 has been replaced by an amino acid selected from the group consisting of Pro, and Tyr.

81. The Factor VII polypeptide according to embodiment 80, wherein said F374 has been replaced by Tyr.

82. The Factor VII polypeptide according to any of embodiments 1-81, wherein the amino acid has been replaced with any other amino acid which can be encoded by polynucleotide constructs.

83. The Factor VII polypeptide according to any of embodiments 1-82, wherein said Fac-tor VII polypeptide is human Factor VII.

84. The Factor VII polypeptide according to any of embodiments 1-83, wherein said Fac-tor VII polypeptide is human Factor VIIa.

85. The Factor VII polypeptide according to any of embodiments 1-84, wherein the ratio between the activity of said Factor VII polypeptide and the activity of the native Factor VIIa polypeptide shown in SEQ ID NO:1 is at least about 1.25.

86. The Factor VII polypeptide according to embodiment 85, wherein said ratio is at least about 2.0, preferably at least about 4.0.

87. The Factor VII polypeptide according to embodiment 1, which is selected from Q176A-FVII, Q176L-FVII L177S-FVII, D196A-FVII, K197A-FVII, K199A-FVII, T238A-FVII, T239I-FVII, T239Y-FVII, Q286A-FVII, D289E-FVII, D289R-FVII, R290Q-FVII, M327Q-FVII, M327N-FVII, K341A-FVII, K341E-FVII, K341Q-FVII, S363M-FVII, S363A-FVII, W364H-FVII, Q366E-FVII.

88. A polynucleotide construct encoding a Factor VII polypeptide according to any of embodiments 1-87.

89. The polynucleotide construct according to embodiment 88, which is a vector.

90. A host cell comprising the polynucleotide construct according to any one of the embodiments 88-89.

91. The host cell according to embodiment 90, which is a eukaryotic cell.

92. The host cell according to embodiment 91, which is of mammalian origin.

93. The host cell according to embodiment 92, wherein the cell is selected from the group consisting of CHO cells , HEK cells and BHK cells.

94. A transgenic animal containing and expressing the polynucleotide construct as defined in embodiment 88.

95. A transgenic plant containing and expressing the polynucleotide construct as defined in embodiment 88.

96. A method for producing the Factor VII polypeptide defined in any of embodiments 1-87, the method comprising cultivating a cell as defined in any one of embodiments 90-93 in an appropriate growth medium under conditions allowing expression of the polynucleotide construct and recovering the resulting polypeptide from the culture medium.

97. A method for producing the Factor VII polypeptide defined in any of embodiments 1-87, the method comprising recovering the Factor VII polypeptide from milk pro-duced by the transgenic animal defined in embodiment 94.

98. A method for producing the Factor VII polypeptide defined in any of embodiments 1-87, the method comprising cultivating a cell of a transgenic plant as defined in embodiment 95, and recovering the Factor VII polypeptide from the plant.

99. A pharmaceutical composition comprising a Factor VII polypeptide comprising one or more substitutions relative to the amino acid sequence of SEQ ID NO:1, wherein said substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO: 1 and wherein said Factor VII polypeptide exhibits increased resistance to inactivation by an endogenous inhibitor of said FVII polypeptide relative to wild-type human FVIIa; and, optionally, a pharmaceutically acceptable carrier.

100. A pharmaceutical composition comprising a Factor VII polypeptide as defined in any of embodiments 1-87, and, optionally, a pharmaceutically acceptable carrier.

101. Use of a Factor VII polypeptide comprising one or more substitutions relative to the amino acid sequence of SEQ ID NO:1, wherein said substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein said Factor VII polypeptide exhibits increased resistance to inactivation by an endogenous inhibitor of said FVII polypeptide relative to wild-type human FVIIa; for the preparation of a medicament for the treatment of bleeding disorders or bleeding episodes or for the enhancement of the normal haemostatic system.

102. Use of a Factor VII polypeptide as defined in any of embodiments 1-87 for the preparation of a medicament for the treatment of bleeding disorders or bleeding episodes or for the enhancement of the normal haemostatic system.

103. Use according to any of embodiments 101-102 for the treatment of haemophilia A or B.

104. A method for the treatment of bleeding disorders or bleeding episodes in a subject or for the enhancement of the normal haemostatic system, the method comprising administering a therapeutically or prophylactically effective amount of a Factor VII polypeptide comprising one or more substitutions relative to the amino acid sequence of SEQ ID NO:1, wherein said substitutions are replacement with any other amino acid of one or more amino acids at a position selected from the group consisting of position 172, 173, 175, 176, 177, 196, 197, 198, 199, 200, 203, 235, 237, 238, 239, 240, 286, 287, 288, 289, 290, 291, 292, 293, 294, 295, 297, 299, 319, 320, 321, 327, 341, 363, 364, 365, 366, 367, 370, 373 corresponding to amino acid positions of SEQ ID NO:1 and wherein said Factor VII polypeptide exhibits increased resistance to inactivation by an endogenous inhibitor of said FVII polypeptide relative to wild-type human FVIIa; to a subject in need thereof.

105. A method for the treatment of bleeding disorders or bleeding episodes in a subject or for the enhancement of the normal haemostatic system, the method comprising administering a therapeutically or prophylactically effective amount of a Factor VII polypeptide as defined in any of embodiments 1-87 to a subject in need thereof.

106. A Factor VII polypeptide as defined in any of embodiments 1-87 for use as a medicament.

107. The Factor VII polypeptide according to any of embodiments 1-106, wherein said Factor VII polypeptide is not a Factor VII polypeptide selected from the list consisting of Q366E-FVII, Q366A-FVII, and Q366G-FVII.

## Claims

1. A Factor VII polypeptide comprising one or more substitutions relative to the amino acid sequence of SEQ ID NO: 1, wherein at least one substitution is a replacement with any other amino acid of the amino acid at position 286 corresponding to amino acid position of SEQ ID NO:1 and wherein said Factor VII polypeptide exhibits increased resistance to inactivation by an endogenous inhibitor of said FVII polypeptide relative to wild-type human FVIIa.

2. The Factor VII polypeptide according to claim 1, wherein said Factor VII polypeptide has increased functional in vivo half-life relative to human wild-type Factor VIIa.

3. The Factor VII polypeptide according to any of claims 1-2, wherein T293 is replaced with any other amino acid.

4. The Factor VII polypeptide according to any of claims 1-3, wherein at least one amino acid in the remaining positions in the protease domain has been replaced with any other amino acid.

5. The Factor VII polypeptide according to any of claims 1-4, wherein V158 has been replaced by an amino acid selected from the group consisting of Ser, Thr, Asn, Gln, Asp, and Glu.

6. The Factor VII polypeptide according to any of claims 1-5 wherein E296 has been replaced by an amino acid selected from the group consisting of Arg, Lys, Ile, Leu and Val.

7. The Factor VII polypeptide according to any of claims 1-6, wherein M298 has been replaced by an amino acid selected from the group consisting of Lys, Arg, Gln, and Asn.

8. The Factor VII polypeptide according to any of claims 1-7, wherein L305 has been replaced by an amino acid selected from the group consisting of Val, Tyr and Ile.

9. The Factor VII polypeptide according to any of claims 1-8, wherein said Factor VII polypeptide is human Factor VIIa.

10. The Factor VII polypeptide according to any of claims 1-9, wherein the ratio between the activity of said Factor VII polypeptide and the activity of the native Factor VIIa polypeptide shown in SEQ ID NO:1 is at least about 1.25.

11. The Factor VII polypeptide according to claim 10, wherein said ratio is at least about 2.0, preferably at least about 4.0.

12. A polynucleotide construct encoding a Factor VII polypeptide according to any of claims 1-11.

13. A host cell comprising the polynucleotide construct according to claim 12.

14. A method for producing the Factor VII polypeptide defined in any of claims 1-11, the method comprising cultivating a cell as defined in claim 13 in an appropriate growth medium under conditions allowing expression of the polynucleotide construct and recovering the resulting polypeptide from the culture medium.

**15.** A pharmaceutical composition comprising a Factor VII polypeptide as defined in any of claims 1-11, and, optionally, a pharmaceutically acceptable carrier.

FIGURE 1 - The amino acid sequence of native human coagulation Factor VII
(SEQ ID NO. 1)


Ala-Asn-Ala-Phe-Leu-GLA-GLA-Leu-Arg-Pro-Gly-Ser-Leu-GLA-Arg-GLA-Cys-Lys-

GLA-GLA-Gln-Cys-Ser-Phe-GLA-GLA-Ala-Arg-GLA-Ile-Phe-Lys-Asp-Ala-GLA-Arg-

Thr-Lys-Leu-Phe-Trp-Ile-Ser-Tyr-Ser-Asp-Gly-Asp-Gln-Cys-Ala-Ser-Ser-Pro-

Cys-Gln-Asn-Gly-Gly-Ser-Cys-Lys-Asp-Gln-Leu-Gln-Ser-Tyr-Ile-Cys-Phe-Cys-

Leu-Pro-Ala-Phe-Glu-Gly-Arg-Asn-Cys-Glu-Thr-His-Lys-Asp-Asp-Gln-Leu-Ile-

Cys-Val-Asn-Glu-Asn-Gly-Gly-Cys-Glu-Gln-Tyr-Cys-Ser-Asp-His-Thr-Gly-Thr-

Lys-Arg-Ser-Cys-Arg-Cys-His-Glu-Gly-Tyr-Ser-Leu-Leu-Ala-Asp-Gly-Val-Ser-

Cys-Thr-Pro-Thr-Val-Glu-Tyr-Pro-Cys-Gly-Lys-Ile-Pro-Ile-Leu-Glu-Lys-Arg-

Asn-Ala-Ser-Lys-Pro-Gln-Gly-Arg-Ile-Val-Gly-Gly-Lys-Val-Cys-Pro-Lys-Gly-

Glu-Cys-Pro-Trp-Gln-Val-Leu-Leu-Leu-Val-Asn-Gly-Ala-Gln-Leu-Cys-Gly-Gly-

Thr-Leu-Ile-Asn-Thr-Ile-Trp-Val-Val-Ser-Ala-Ala-His-Cys-Phe-Asp-Lys-Ile-

Fig. 1 (continued)

Lys-Asn-Trp-Arg-Asn-Leu-Ile-Ala-Val-Leu-Gly-Glu-His-Asp-Leu-Ser-Glu-His-
200                205                210                215

Asp-Gly-Asp-Glu-Gln-Ser-Arg-Arg-Val-Ala-Gln-Val-Ile-Ile-Pro-Ser-Thr-Tyr-
220                225                230

Val-Pro-Gly-Thr-Thr-Asn-His-Asp-Ile-Ala-Leu-Leu-Arg-Leu-His-Gln-Pro-Val-
235                240                245                250

Val-Leu-Thr-Asp-His-Val-Val-Pro-Leu-Cys-Leu-Pro-Glu-Arg-Thr-Phe-Ser-Glu-
255                260                265                270

Arg-Thr-Leu-Ala-Phe-Val-Arg-Phe-Ser-Leu-Val-Ser-Gly-Trp-Gly-Gln-Leu-Leu-
275                280                285

Asp-Arg-Gly-Ala-Thr-Ala-Leu-Glu-Leu-Met-Val-Leu-Asn-Val-Pro-Arg-Leu-Met-
290                295                300                305 306

Thr-Gln-Asp-Cys-Leu-Gln-Gln-Ser-Arg-Lys-Val-Gly-Asp-Ser-Pro-Asn-Ile-Thr-
310                315                320

Glu-Tyr-Met-Phe-Cys-Ala-Gly-Tyr-Ser-Asp-Gly-Ser-Lys-Asp-Ser-Cys-Lys-Gly-
325                330                335                340

Asp-Ser-Gly-Gly-Pro-His-Ala-Thr-His-Tyr-Arg-Gly-Thr-Trp-Tyr-Leu-Thr-Gly-
345                350                355                360

Ile-Val-Ser-Trp-Gly-Gln-Gly-Cys-Ala-Thr-Val-Gly-His-Phe-Gly-Val-Tyr-Thr-
365                370                375

Arg-Val-Ser-Gln-Tyr-Ile-Glu-Trp-Leu-Gln-Lys-Leu-Met-Arg-Ser-Glu-Pro-Arg-
380                385                390                395

Pro-Gly-Val-Leu-Leu-Arg-Ala-Pro-Phe-Pro
400                405 406

FIGURE 2 - Illustrates of activity upon ATIII inhibition (relative to WT FVIIa) plotted against the FX activation data (relative to WT FVIIa) for FVIIa mutants generated according to example 7.

## EUROPEAN SEARCH REPORT

Application Number

EP 10 18 1064

**DOCUMENTS CONSIDERED TO BE RELEVANT**

| Category | Citation of document with indication, where appropriate, of relevant passages | Relevant to claim | CLASSIFICATION OF THE APPLICATION (IPC) |
|---|---|---|---|
| X | WO 2004/029091 A (MAXYGEN APS; MAXYGEN HOLDINGS LTD; HAANING, JESPER, MORTENSEN; ANDERSE) 8 April 2004 (2004-04-08) <br> * page 31, lines 3-7; claims 47,48 * <br> * page 41, line 6 * <br> ----- | 1-15 | INV. <br> C12N9/64 <br> A61K38/36 |
| X | WO 01/58935 A2 (MAXYGEN APS [DK]) 16 August 2001 (2001-08-16) <br> * page 27, line 21 * <br> * page 30, line 1 * <br> ----- | 1-4,9-15 | |

**TECHNICAL FIELDS SEARCHED (IPC)**

C12N
A61K

The present search report has been drawn up for all claims

| Place of search | Date of completion of the search | Examiner |
|---|---|---|
| Munich | 17 December 2010 | Tudor, Mark |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&: member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 18 1064

This annex lists the patent family members relating to the patent documents cited in the above-mentioned European search report.
The members are as contained in the European Patent Office EDP file on
The European Patent Office is in no way liable for these particulars which are merely given for the purpose of information.

17-12-2010

| Patent document cited in search report | | Publication date | Patent family member(s) | | Publication date |
|---|---|---|---|---|---|
| WO 2004029091 | A | 08-04-2004 | AU | 2003266931 A1 | 19-04-2004 |
| | | | AU | 2010201266 A1 | 22-04-2010 |
| | | | CA | 2502162 A1 | 08-04-2004 |
| | | | EP | 1549677 A2 | 06-07-2005 |
| | | | JP | 4472526 B2 | 02-06-2010 |
| | | | JP | 2006517089 T | 20-07-2006 |
| WO 0158935 | A2 | 16-08-2001 | AT | 428445 T | 15-05-2009 |
| | | | AU | 783512 B2 | 03-11-2005 |
| | | | AU | 2006200448 A1 | 02-03-2006 |
| | | | AU | 2010200296 A1 | 18-02-2010 |
| | | | CA | 2397347 A1 | 16-08-2001 |
| | | | CN | 1400910 A | 05-03-2003 |
| | | | DK | 1257295 T3 | 10-08-2009 |
| | | | EP | 1257295 A2 | 20-11-2002 |
| | | | EP | 1982732 A2 | 22-10-2008 |
| | | | ES | 2325877 T3 | 23-09-2009 |
| | | | JP | 2003521930 T | 22-07-2003 |
| | | | JP | 4509846 B2 | 21-07-2010 |
| | | | JP | 2005270110 A | 06-10-2005 |
| | | | NO | 20023804 A | 25-09-2002 |
| | | | NZ | 521257 A | 29-10-2004 |
| | | | PL | 362856 A1 | 02-11-2004 |
| | | | PL | 206148 B1 | 30-07-2010 |
| | | | PT | 1257295 E | 16-07-2009 |
| | | | RU | 2278123 C2 | 20-06-2006 |
| | | | US | 2007243588 A1 | 18-10-2007 |
| | | | US | 2006252127 A1 | 09-11-2006 |
| | | | US | 2006258585 A1 | 16-11-2006 |
| | | | US | 2006228782 A1 | 12-10-2006 |
| | | | US | 2006240524 A1 | 26-10-2006 |
| | | | US | 2006240525 A1 | 26-10-2006 |
| | | | US | 2006252689 A1 | 09-11-2006 |
| | | | US | 2006252690 A1 | 09-11-2006 |
| | | | US | 2007142280 A1 | 21-06-2007 |
| | | | US | 2003096338 A1 | 22-05-2003 |
| | | | US | 2009023635 A1 | 22-01-2009 |
| | | | US | 2006019336 A1 | 26-01-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 200421 A
- US 4784950 A
- WO 0331464 A
- US 20040043446 A
- US 20040063911 A
- US 20040142856 A
- US 20040137557 A
- US 20040132640 A
- WO 0104287 A
- US 20030165996 A
- WO 0158935 A
- WO 0393465 A
- WO 0202764 A
- US 20030211094 A
- US 5580560 A
- WO 02077218 A
- WO 0238162 A
- WO 9920767 A
- US 6017882 A
- US 6747003 B
- US 20030100506 A
- WO 0066753 A
- US 20010018414 A
- US 2004220106 A
- US 200131005 B
- US 6762286 B
- US 6693075 B
- US 6806063 B
- US 20030096338 A
- WO 04029091 A
- WO 04083361 A
- WO 04111242 A
- WO 04108763 A
- WO 0183725 A
- WO 0222776 A
- WO 03027147 A
- WO 04029090 A
- JP 2001061479 B
- US 4683202 A
- US 5288629 A
- US 4745051 A
- EP 397485 A
- US 5155037 A
- US 5162222 A
- US 4599311 A
- EP 238023 A
- EP 383779 A
- US 4870008 A
- WO 8702670 A
- US 4546082 A
- EP 16201 A
- EP 123294 A
- EP 123544 A
- EP 163529 A
- WO 8902463 A
- WO 9211378 A
- EP 215594 A
- WO 9005783 A
- US 5023328 A
- US 4713339 A, Levinson and Simonsen
- US 4931373 A
- US 5037743 A
- US 4845075 A
- US 4882279 A
- EP 272277 A
- EP 184438 A
- EP 244234 A
- US 4879236 A
- US 5077214 A
- WO 8901029 A
- WO 8901028 A
- WO 8800239 A
- US 5304489 A
- WO 8901343 A
- WO 9102318 A
- US 4873316 A, Meade
- US 4873191 A
- WO 9005188 A
- WO 9211757 A
- GB 8700458 A
- EP 0255378 A
- US 4456591 A
- US 4837028 A
- US 4501728 A
- US 4975282 A

**Non-patent literature cited in the description**

- **Dickinson et al.** *Proc. Natl. Acad. Sci. USA,* 1996, vol. 93, 14379-14384
- **Shobe et al.** *J.Biol.Chem.,* 1999, vol. 274, 24171-24175

- **Baugh et al.** *J.Biol.Chem.,* 2000, vol. 275, 28826-28833
- **Lino et al.** *Arch. Biochem. Biophys.,* 1998, vol. 352, 182-192
- **Mollerup et al.** *Biotechnol. Bioeng.,* 1995, vol. 48, 501-505
- **Kornfelt et al.** *Arch. Biochem. Biophys.,* 1999, vol. 363, 43-54
- **Graham et al.** *J. Gen. Virol.,* 1977, vol. 36, 59-72
- **Waechter ; Baserga.** *Proc.Natl.Acad.Sci.USA,* 1982, vol. 79, 1106-1110
- **Urlaub ; Chasin.** *Proc. Natl. Acad. Sci. USA,* 1980, vol. 77, 4216-4220
- **Takeya et al.** *J. Biol. Chem.,* 1988, vol. 263, 14868-14872
- **Zoller ; Smith.** *DNA,* 1984, vol. 3, 479-488
- **Horton et al.** Splicing by extension overlap. *Gene,* 1989, vol. 77, 61-68
- PCR Protocols. Academic Press, 1990
- **Sambrook et al.** Molecular Cloning: A Laboratory Manual. Cold Spring Harbor Laboratory, Cold Spring Harbor, 1989
- **Beaucage ; Caruthers.** *Tetrahedron Letters,* 1981, vol. 22, 1859-1869
- **Matthes et al.** *EMBO Journal,* 1984, vol. 3, 801-805
- **Saiki et al.** *Science,* 1988, vol. 239, 487-491
- **Subramani et al.** *Mol. Cell Biol.,* 1981, vol. 1, 854-864
- **Palmiter et al.** *Science,* 1983, vol. 222, 809-814
- **Boshart et al.** *Cell,* 1985, vol. 41, 521-530
- **Kaufman ; Sharp.** *Mol. Cell. Biol,* 1982, vol. 2, 1304-1319
- **Vasuvedan et al.** *FEBS Lett.,* 1992, vol. 311, 7-11
- **J.M. Vlak et al.** *J. Gen. Virology,* 1988, vol. 69, 765-776
- **Hitzeman et al.** *J. Biol. Chem.,* 1980, vol. 255, 12073-12080
- **Alber ; Kawasaki.** *J. Mol. Appl. Gen.,* 1982, vol. 1, 419-434
- **Young et al.** Genetic Engineering of Microorganisms for Chemicals. Plenum Press
- **Russell et al.** *Nature,* 1983, vol. 304, 652-654
- **McKnight et al.** *The EMBO J.,* 1985, vol. 4, 2093-2099
- **DeNoto et al.** *Nucl. Acids Res.,* 1981, vol. 9, 3719-3730
- **O. Hagenbuchle et al.** *Nature,* 1981, vol. 289, 643-646
- **L.A. Valls et al.** *Cell,* 1987, vol. 48, 887-897
- **M. Egel-Mitani et al.** *Yeast,* 1990, vol. 6, 127-137
- **Sambrook et al.** Molecular Cloning: A Laboratory Manual. Cold Spring Harbor, 1989
- **Kaufman ; Sharp.** *J. Mol. Biol.,* 1982, vol. 159, 601-621
- **Southern ; Berg.** *J. Mol. Appl. Genet.,* 1982, vol. 1, 327-341
- **Loyter et al.** *Proc. Natl. Acad. Sci. USA,* 1982, vol. 79, 422-426
- **Wigler et al.** *Cell,* 1978, vol. 14, 725
- **Corsaro ; Pearson.** *Somatic Cell Genetics,* 1981, vol. 7, 603
- **Graham ; van der Eb.** *Virology,* 1973, vol. 52, 456
- **Neumann et al.** *EMBO J.,* 1982, vol. 1, 841-845
- **Wigler et al.** *Cell,* 1978, vol. 14, 725-732
- **Corsaro ; Pearson.** *Somatic Cell Genetics,* 1981, vol. 7, 603-616
- **Graham ; Van der Eb.** *Virology,* 1973, vol. 52d, 456-467
- **Waechter ; Baserga.** *Proc. Natl. Acad. Sci. USA,* 1982, vol. 79, 1106-1110
- **Gleeson et al.** *Gen. Microbiol.,* 1986, vol. 132, 3459-3465
- **Malardier et al.** *Gene,* 1989, vol. 78, 147-156
- **Whitelaw et al.** *Biochem. J.,* 1992, vol. 286, 31-39
- **Brinster et al.** *Proc. Natl. Acad. Sci. USA,* 1988, vol. 85, 836-840
- **Palmiter et al.** *Proc. Natl. Acad. Sci. USA,* 1991, vol. 88, 478-482
- **Whitelaw et al.** *Transgenic Res.,* 1991, vol. 1, 3-13
- **von Heijne.** *Nucl. Acids Res.,* 1986, vol. 14, 4683-4690
- **Jaenisch.** *Science,* 1988, vol. 240, 1468-1474
- **Bradley et al.** *Bio/Technology,* 1992, vol. 10, 534-539
- **Hogan et al.** Manipulating the Mouse Embryo: A Laboratory Manual. Cold Spring Harbor Laboratory, 1986
- **Simons et al.** *Bio/Technology,* 1988, vol. 6, 179-183
- **Wall et al.** *Biol. Reprod.,* 1985, vol. 32, 645-651
- **Buhler et al.** *Bio/Technology,* 1990, vol. 8, 140-143
- **Ebert et al.** *Bio/Technology,* 1991, vol. 9, 835-838
- **Krimpenfort et al.** *Bio/Technology,* 1991, vol. 9, 844-847
- **Wall et al.** *J. Cell. Biochem.,* 1992, vol. 49, 113-120
- **Gordon et al.** *Proc. Natl. Acad. Sci. USA,* 1980, vol. 77, 7380-7384
- **Gordon ; Ruddle.** *Science,* 1991, vol. 214, 1244-1246
- **Palmiter ; Brinster.** *Cell,* 1985, vol. 41, 343-345

- **Brinster et al.** *Proc. Natl. Acad. Sci. USA,* 1985, vol. 82, 4438-4442
- **Hogan et al.** *PROC. NATL. ACAD. SCI. USA*
- **Hiatt.** *Nature,* 1990, vol. 344, 469-479
- **Edelbaum et al.** *J. Interferon Res.,* 1992, vol. 12, 449-453
- **Sijmons et al.** *Bio/Technology,* 1990, vol. 8, 217-221
- Protein Purification. VCH Publishers, 1989
- **Wakabayashi et al.** *J. Biol. Chem.,* 1986, vol. 261, 11097-11108
- **Thim et al.** *Biochemistry,* 1988, vol. 27, 7785-7793
- **Scopes, R.** Protein Purification. Springer-Verlag, 1982
- **Osterud et al.** *Biochemistry,* 1972, vol. 11, 2853-2857
- **Hedner ; Kisiel.** *J. Clin. Invest.,* 1983, vol. 71, 1836-1841
- **Kisiel ; Fujikawa.** *Behring Inst. Mitt.,* 1983, vol. 73, 29-42
- **Bjoern et al.** *Research Disclosure,* September 1986, vol. 269, 564-565
- **Monroe et al.** *Brit. J. Haematol.,* 1997, vol. 99, 542-547
- Remington's Pharmaceutical Sciences. Mack Publishing Company, 1990